# EUROPEAN PATENT SPECIFICATION

(11) **EP 0 735 019 B1**
(45) Date of publication and mention of the grant of the patent: **20.09.2000**
(21) Application number: 96107357.4
(22) Date of filing: 18.11.1991
(51) Int. Cl.: C07C 271/22, C07D 215/48, A61K 31/325, A61K 31/47

(54) **Retroviral protease inhibitors**
Inhibitoren retroviraler Proteasen
Inhibiteurs de protéases rétrovirales

(30) Priority: 19.11.1990 US 615210; 14.11.1991 US 789643
(43) Date of publication of application: 02.10.1996
(62) Divisional of application: 92901691.3
(73) Proprietor: MONSANTO COMPANY, St. Louis, Missouri 63167 (US)
(72) Inventor: Reed, Kathryn Lea, Richmond Heights, Missouri 63117 (US); Talley, John Jeffrey, Chesterfield, Missouri 63017 (US)
(74) Representative: Bosch, Henry

(56) References cited:
- EP-A- 0 172 347
- EP-A- 0 223 437
- FR-A- 2 620 451
- JOURNAL OF MEDICINAL CHEMISTRY, vol. 30, no. 7, July 1987, WASHINGTON, DC, US, pages 1224-1228, XP002027217 S.H. ROSENBERG, ET AL.: "Novel renin inhibitors containing analogues of statine retro-inverted at the C-termini: specificity at the P2 histidine site"

## Description

### 1. Field of the Invention

The present invention relates to retroviral protease inhibitors and, more particularly, relates to novel compounds and to use of the compounds for the manufacture of a medicament for inhibiting retroviral proteases. This invention, in particular, relates to hydroxyethylamine protease inhibitor compounds, and the use of the compounds for the manufacture of a medicament for inhibiting retroviral proteases such as human immunodeficiency virus (HIV) protease and for treating a retroviral infection, e.g., an HIV infection.

### 2. Related Art

During the replication cycle of retroviruses, gag and gag-pol gene products are translated as proteins. These proteins are subsequently processed by a virally encoded protease (or proteinase) to yield viral enzymes and structural proteins of the virus core. Most commonly, the gag precursor proteins are processed into the core proteins and the pol precursor proteins are processed into the viral enzymes, e.g., reverse transcriptase and retroviral protease. It has been shown that correct processing of the precursor proteins by the retroviral protease is necessary for assembly of infectious virons. For example, it has been shown that frameshift mutations in the protease region of the pol gene of HIV prevents processing of the gag precursor protein. It has also been shown through site-directed mutagenesis of an aspartic acid residue in the HIV protease that processing of the gag precursor protein is prevented. Thus, attempts have been made to inhibit viral replication by inhibiting the action of retroviral proteases.

Retroviral protease inhibition typically involves a transition-state mimetic whereby the retroviral protease is exposed to a mimetic compound which binds (typically in a reversible manner) to the enzyme in competition with the gag and gag-pol proteins to thereby inhibit replication of structural proteins and, more importantly, the retroviral protease itself. In this manner, retroviral replication proteases can be effectively inhibited.

Several classes of mimetic compounds have been proposed, particularly for inhibition of proteases, such as for inhibition of HIV protease. Such mimetics include hydroxyethylamine isosteres and reduced amide isosteres. See, for example, EP O 346 847; EP O 342,541; Roberts et al, "Rational Design of Peptide-Based Proteinase Inhibitors, "Science, 248, 358 (1990); and Erickson et al, "Design Activity, and 2.8A Crystal Structure of a C₂ Symmetric Inhibitor Complexed to HIV-1 Protease," Science, 249, 527 (1990).

Several classes of mimetic compounds are known to be useful as inhibitors of the proteolytic enzyme renin. See, for example, U.S. No. 4,599,198; U.K. 2,184,730; G.B. 2,209,752; EP O 264 795; G.B. 2,200,115 and U.S. SIR H725. However, it is known that, although renin and HIV proteases are both classified as aspartyl proteases, compounds which are effective renin inhibitors generally cannot be predicted to be effective HIV protease inhibitors.

### BRIEF DESCRIPTION OF THE INVENTION

The present invention is directed to virus inhibiting compounds and compositions. More particularly, the present invention is directed to retroviral protease inhibiting compounds and pharmaceutical composition comprising the novel compound exhibiting retroviral protease inhibiting properties and to the use of the novel compounds for the manufacture of a medicament.

### DETAILED DESCRIPTION OF THE INVENTION

In accordance with the present invention, there is provided a compound represented by the formula 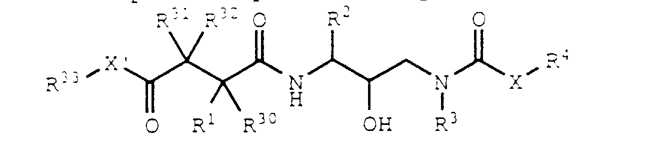 or a pharmaceutically acceptable salt thereof, wherein R¹ represents hydrogen, alkyl of 1 to 4 carbon atoms, aralkyl or hydroxyl radicals;
R³⁰, R³¹ and R³² represent radicals as defined for R¹, or one of R¹ and R³⁰ together with one of R³¹ and R³² and the carbon atoms to which they are attached form a cycloalkyl radical; or R³⁰ and R³² together with the carbon atoms to which they are attached form a three to six-membered cycloalkyl radical;
R² represents alkyl, aryl, cycloalkyl, cycloalkylalkyl or aralkyl radicals, which radicals are optionally substituted with halogen, -OR⁹ or SR⁹ radicals, wherein R⁹ represents hydrogen or alkyl radicals;
X represents O or CH(R¹⁷), wherein R¹⁷ represents hydrogen or alkyl radicals;
R³ represents alkyl radical of 2 to 5 carbon atoms and R⁴ represents alkyl or cycloalkyl radicals; or R³ represents heteroaralkyl, alkyl of 2 to 5 carbon atoms, benzyl, parafluorobenzyl, para-methoxybenzyl, para-methylbenzyl, 2-naphthylmethyl or cyclohexylmethyl radicals and R⁴ represents t-butyl; or provided X represents CHR¹⁷, R³ and R⁴ independently represent alkyl or hydroxyalkyl radicals;
X' represents NR³⁴ or O; and
R³³ and R³⁴ independently represent hydrogen and radicals as defined for R³; or provided X' represents NR³⁴, R³³ and R³⁴ together with a nitrogen atom to which they are bonded represent heterocyclyl or heteroaryl radicals; or provided X' represents O, R³³ represents hydrogen, alkyl or aralkyl radicals; and
wherein alkyl, alone or in combination, is a straight-chain or branched-chain alkyl radical containing from 1 to 8 carbon atoms; cycloalkyl, alone or in combination, is an cyclic alkyl radical containing from 3 to 8 carbon atoms; aryl, alone or in combination, is an unsubstituted phenyl or naphthyl radical, or phenyl or naphthyl radical substituted with a C₁-C₈ alkyl, C₁-C₈ alkoxy, halogen, hydroxy or amino radical; heterocycloalkyl, alone or in combination, is pyrrolidinyl, piperidinyl, piperazinyl, morpholinyl, thiamorpholinyl, tetrahydroquinolinyl or tetrahydroisoquinolinyl radicals, which radicals are optionally substituted on a substitutable carbon atom with halogen, C₁-C₈ alkyl, C₁-C₈ alkoxy or oxo, on a ring secondary nitrogen atom with C₁-C₈ alkyl, aryl-C₁-C₈-alkoxycarbonyl, C₁-C₈ alkanoyl, phenyl or phenyl-C₁-C₈-alkyl, or on a ring tertiary nitrogen atom by oxido; and heteroaryl, alone or in combination, is pyrrolyl, imidazolyl, pyrazolyl, pyridyl, pyrazinyl, pyrimidinyl, furyl, thienyl, triazolyl, oxazolyl, thiazolyl, indolyl, quinolinyl, isoquinolinyl, quinoxalinyl, β-carbolinyl, benzofuranyl or benzimidazolyl radicals, which radicals are optionally substituted on a substitutable carbon atom with halogen, C₁-C₈ alkyl, C₁-C₈ alkoxy or oxo, on a ring secondary nitrogen atom with C₁-C₈ alkyl, aryl-C₁-C₈-alkoxycarbonyl, C₁-C₈ alkanoyl, phenyl or phenyl-C₁-C₈-alkyl, or on a ring tertiary nitrogen atom by oxido. 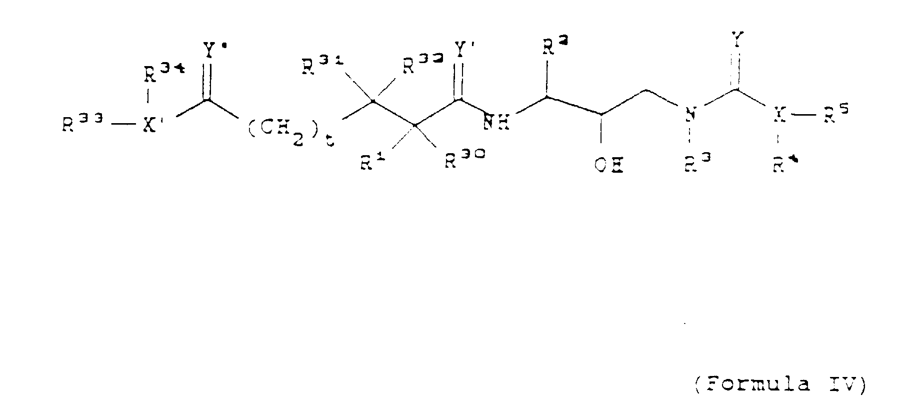 or a pharmaceutically acceptable salt, prodrug or ester thereof wherein X, X' , R¹, R², R³, R⁴, R⁵, R³⁰, R³¹, R³², and R³³ are as defined above.

In the presently claimed compounds, t = O, R³⁴ is hydrogen, and each of Y, Y' and Y'' is oxygen.

As utilized herein, the term "alkyl", alone or in combination, means a straight-chain or branched-chain alkyl radical containing 1 to 10, preferably from 1 to 8, carbon atoms. Examples of such radicals include methyl, ethyl, n-propyl, isopropyl, n-butyl, isobutyl, sec-butyl, tert-butyl, pentyl, isoamyl, hexyl and octyl. The term "alkoxy", alone or in combination, means an alkyl ether radical wherein the term alkyl is as defined above. Examples of suitable alkyl ether radicals include methoxy, ethoxy, n-propoxy, isopropoxy, n-butoxy, iso-butoxy, sec-butoxy, and tert-butoxy. The term "cycloalkyl" means an alkyl radical which contains from 3 to 8 carbon atoms and is cyclic. The term "cycloalkylalkyl" means an alkyl radical as defined above which is substituted by a cycloalkyl radical containing from 3 to 8, preferably from 3 to 6, carbon atoms. Examples of such cycloalkyl radicals include cyclopropyl, cyclobutyl, cyclopentyl and cyclohexyl. The term "aryl", alone or in combination, means a phenyl or naphthyl radical which optionally carries one or more substituents selected from alkyl, alkoxy, halogen, hydroxy and amino such as phenyl, p-tolyl, 4-methoxyphenyl, 4-(tert-butoxy)phenyl, 4-fluorophenyl, 4-chlorophenyl, 4-hydroxyphenyl, 1-naphthyl and 2-naphthyl. The term "aralkyl", alone or in combination, means an alkyl radical as defined above in which one hydrogen atom is replaced by an aryl radical as defined above, such as benzyl and 2-phenylethyl. The term "aralkoxy carbonyl", alone or in combination, means a radical of the formula -C(O)-O-aralkyl in which the term "aralkyl" has the significance given above. An example of an aralkoxycarbonyl radical is benzyloxycarbonyl. The term "aryloxy" means a radical of the formula aryl-o- in which the term aryl has the significance given above. The term "alkanoyl", alone or in combination, means an acyl radical derived from an alkanecarboxylic acid, examples of which include acetyl, propionyl, butyryl, valeryl and 4-methylvaleryl. The term "cycloalkylcarbonyl" means an acyl group derived from a monocyclic or bridged cycloalkanecarboxylic acid such as cyclopropanecarbonyl, cyclohexanecarbonyl and adamantanecarbonyl, or from a benz-fused monocyclic cycloalkanecarboxylic acid which is optionally substituted by, for example, alkanoylamino, such as 1,2,3,4-tetrahydro-2-naphthoyl,2-acetamido-1,2,3,4-tetrahydro-2-naphthcyl. The term "aralkanoyl" means an acyl radical derived from an aryl-substituted alkanecarboxylic acid such as phenylacetyl, 3-phenylpropionyl (hydrocinnamoyl), 4-phenylbutyryl, (2-naphthyl)acetyl, 4-chlorohydrocinnamoyl, 4-aminohydroinnamoyl and 4-methoxyhydrocinnamoyl. The term "aroyl" means an acyl radical derived from an aromatic carboxylic acid. Examples of such radicals include aromatic carboxylic acids, an optionally substituted benzoic or naphthoic acid such as benzoyl, 4-chlorobenzoyl, 4-carboxybenzoyl, 4-(benzyloxycarbonyl)benzoyl, 1-naphthoyl, 2-naphthoyl, 5-carboxy-2 naphthoyl, 6-(benzyloxycarbonyl)-2-naphthoyl, 3-benzyloxy-2-naphthoyl, 3-hydroxy-2-naphthoyl and 3-(benzyloxyformamido)-2-naphthoyl. The heterocyclyl or heterocycloalkyl portion of a heterocyclylcarbonyl, heterocyclyloxycarbonyl, heterocyclylalkoxycarbonyl, or heterocyclyalkyl group or is a saturated or partially unsaturated monocyclic, bicyclic or tricyclic heterocycle which contains one or more hetero atoms selected from nitrogen, oxygen and sulphur, which is optionally substituted on one or more carbon atoms by halogen, alkyl, alkoxy and oxo, and/or on a secondary nitrogen atom (i.e., -NH-) by alkyl, aralkoxycarbonyl, alkanoyl, phenyl or phenylalkyl or on a tertiary nitrogen atom (i.e. = N-) by oxido and which is attached via a carbon atom. The heteroaryl portion of a heteroaroyl, heteroaryloxycarbonyl, or a heteroaralkoxy carbonyl group or is an aromatic monocyclic, bicyclic, or tricyclic heterocycle which contains the hetero atoms and is optionally substituted as defined above with respect to the definition of heterocyclyl. Examples of such heterocyclyl and heteroaryl groups are pyrrolidinyl, piperidinyl, piperazinyl, morpholinyl, thiamorpholinyl, pyrrolyl, imidazolyl (e.g., imidazol 4-yl, 1-benzyloxycarbonylimidazol-4-yl, etc.), pyrazolyl, pyridyl, pyrazinyl, pyrimidinyl, furyl, thienyl, triazolyl, oxazolyl, thiazolyl, indolyl (e.g., 2-indolyl), quinolinyl, (e.g., 2-quinolinyl, 3-quinolinyl, 1-oxido-2-quinolinyl), isoquinolinyl (e.g., 1-isoquinolinyl, 3-isoquinolinyl), tetrahydroquinolinyl (e.g., 1,2,3,4-tetrahydro-2-quinolyl), 1,2,3,4-tetrahydroisoquinolinyl (e.g., 1,2,3,4-tetrahydro-1-oxo-isoquinolinyl), quinoxalinyl, β-carbolinyl, benzofurancarbonyl, and benzimidazolyl radicals. The term "cycloalkylalkoxycarbonyl" means an acyl group derived from a cycloalkylalkoxycarboxylic acid of the formula cycloalkylalkyl-O-COOH wherein cycloalkylalkyl has the significance given above. The term "aryloxyalkanoyl" means an acyl radical of the formula aryl-O-alkanoyl wherein aryl and alkanoyl have the significance given above. The term "heterocyclyloxycarbonyl" means an acyl group derived from heterocyclyl-O-COOH wherein heterocyclyl is as defined above. The term "heterocyclylalkanoyl" is an acyl radical derived from a heterocyclyl-substituted alkane carboxylic acid wherein heterocyclyl has the significance given above. The term "heterocyclylalkoxycarbonyl" means an acyl radical derived from a heterocyclyl-substituted alkane-O-COOH wherein heterocyclyl has the significance given above. The term "heteroaryloxycarbonyl" means an acyl radical derived from a carboxylic acid represented by heteroaryl-O-COOH wherein heteroaryl has the significance given above. The term "aminoalkanoyl" means an acyl group derived from an amino-substituted alkanecarboxylic acid wherein the amino group can be a primary, secondary or tertiary amino group containing substituents selected from hydrogen, and alkyl, aryl, aralkyl, cycloalkyl and cycloalkylalkyl radicals. The term "halogen" means fluorine, chlorine, bromine or iodine. The term "leaving group" generally refers to groups readily displaceable by a nucleophile, such as an amine, a thiol or an alcohol nucleophile. Such leaving groups are well known and include carboxylates, N-hydroxysuccinimide, N-hydroxybenzotriazole, halides, triflates, tosylates -OR and -SR. Preferred leaving groups are indicated herein where appropriate.

Procedures for preparing the compounds of Formula I are set forth below. It should be noted that the general procedure is shown as it relates to preparation of compounds having the specified stereochemistry, for example, wherein the stereochemistry about the hydroxyl group is designated as (R). However, such procedures are generally applicable, as illustrated, to those compounds of opposite configuration, e.g., where the stereochemistry about the hydroxyl group is (S).

### Preparation of Compounds of Formula

To produce claimed compounds, starting with a lactate of the formula: 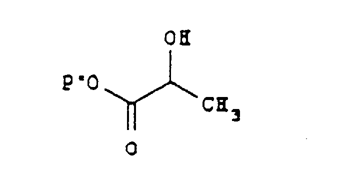 wherein P" represents alkyl radicals, such as, for example, ethyl, methyl, benzyl and the like. The hydroxyl group of the lactate is protected as its ketal by reaction in a suitable solvent system with methyl isopropenyl ether (1,2-methoxypropene) in the presence of a suitable acid. Suitable solvent systems include methylene chloride, tetrahydrofuran and the like as well as mixtures thereof. Suitable acids include POCl₃ and the like. It should be noted that well-known groups other than methyl isopropenyl ether can be utilized to form the ketal. The ketal is then reduced with diisobutylaluminum hydride (DIBAL) at -78°C to produce the corresponding aldehyde which is then treated with ethylidene triphenylphosphorane (Wittig reaction) to produce a compound represented by the formula:

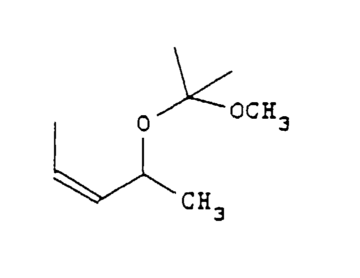

The ketal protecting group is then removed utilizing procedures well-known in the art such as by mild acid hydrolysis. The resulting compound is then esterified with isobutyryl chloride to produce a compound of the formula:

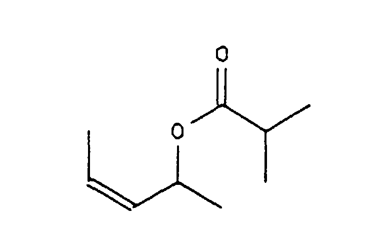

This compound is then treated with lithium diisopropyl amide at -78°C followed by warming of the reaction mixture to room temperature to effect a Claisen rearrangement ([3,3]) to produce the corresponding acid represented by the formula:

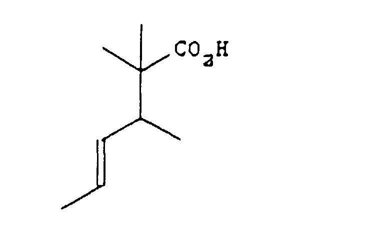

Treatment of the acid with benzyl bromide in the presence of a tertiary amine base, e.g., DBU, produces the corresponding ester which is then cleaved oxidatively to give a trisubstituted succinic acid:

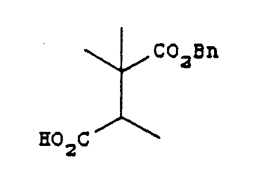

The trisubstituted succinic acid is then coupled to the urea isostere as described above. To produce the free acid, the benzyl ester is removed by hydrogenolysis to produce the corresponding acid. The acid can then be converted to the primary amide by methods well-known in the art.

An alternative method for preparing trisubstituted succinic acids involves reacting an ester of acetoacetic acid represented by the formula: 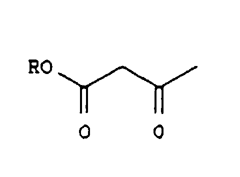 where R is a suitable protecting group, such as methyl, ethyl, benzyl or t-butyl with sodium hydride and a hydrocarbyl halide (R³¹X or R³²X) in a suitable solvent, e.g., THF, to produce the corresponding disubstituted derivative represented by the formula: 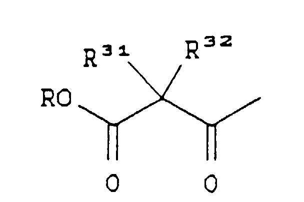 This disubstituted acetoacetic acid derivative is then treated with lithium diisopropyl amide at about -10°C and in the presence of PhN(triflate)₂ to produce a vinyl triflate of the formula:

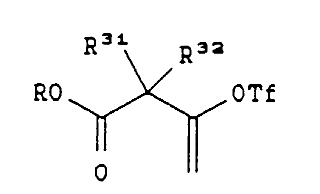

The vinyl triflate is then carbonylated utilizing a palladium catalyst, e.g., Pd₂(OAc)(Ph₃)P, in the presence of an alcohol (R"OH) or water (R"=H) and a base, e.g., triethylamine, in a suitable solvent such as DMF, to produce the olefinic ester or acid of the formula: 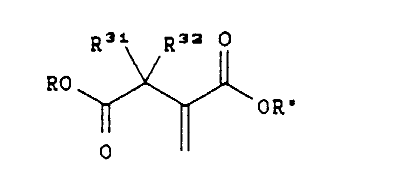 The olefin can then be subsequently asymmetrically hydrogenated, as described above, to produce a trisubstituted succinic acid derivative of the formula: 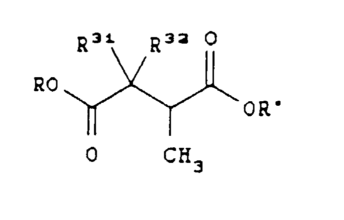 If R" is not H, the ester group can be removed either by hydrolysis, acidolysis, or hydrogenolysis, and the corresponding acid is then coupled to the amino alcohol derivatives as described above and then, optionally, the R group removed to produce the corresponding acid, and optionally, converted to the amide.

Alternatively, one can react the amino alcohol derivatives with either a suitably monoprotected succinic acid or glutaric acid of the following structure; 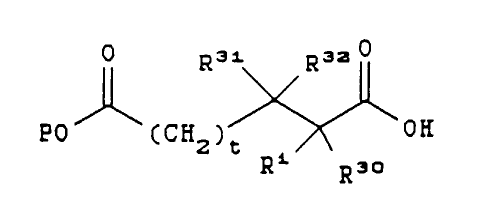 followed by removal of the protecting group and conversion of the resulting acid to an amide. One can also react an anhydride of the following structure; 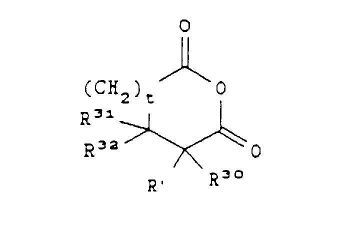 with the amino alcohol derivatives and then separate any isomers or convert the resulting acid to an amide and then separate any isomers.

It is contemplated that for preparing compounds of the Formulas having R⁶, the compounds can be prepared following the procedure set forth above and, prior to coupling the urea derivative or analog thereof to an amino acid, e.g., PNH(CH₂)ₜCH(R¹)COOH, carried through a procedure referred to in the art as reductive amination. Thus, a sodium cyanoborohydride and an appropriate aldehyde R⁶C(O)H or ketone R⁶C(O)R⁶ can be reacted with the urea derivative compound or appropriate analog at room temperature in order to reductively aminate any of the compounds of Formulas I-IV. It is also contemplated that where R³ of the amino alcohol intermediate is hydrogen, the inhibitor compounds can be prepared through reductive amination of the final product of the reaction between the amino alcohol and the amine or at any other stage of the synthesis for preparing the inhibitor compounds.

Contemplated equivalents of the general formulas set forth above for the antiviral compounds and derivatives as well as the intermediates are compounds otherwise corresponding thereto and having the same general properties wherein one or more of the various R groups are simple variations of the substituents as defined therein, e.g., wherein R is a higher alkyl group than that indicated. In addition, where a substituent is designated as, or can be, a hydrogen, the exact chemical nature of a substituent which is other than hydrogen at that position, e.g., a hydrocarbyl radical or a halogen, hydroxy, amino and the like functional group, is not critical so long as it does not adversely affect the overall activity and/or synthesis procedure.

The chemical reactions described above are generally disclosed in terms of their broadest application to the preparation of the compounds of this invention. Occasionally, the reactions may not be applicable as described to each compound included within the disclosed scope. The compounds for which this occurs will be readily recognized by those skilled in the art. In all such cases, either the reactions can be successfully performed by conventional modifications known to those skilled in the art, e.g., by appropriate protection of interfering groups, by changing to alternative conventional reagents, by routine modification of reaction conditions, or other reactions disclosed herein or otherwise conventional, will be applicable to the preparation of the corresponding compounds of this invention. In all preparative methods, all starting materials are known or readily preparable from known starting materials.

Without further elaboration, it is believed that one skilled in the art can, using the preceding description, utilize the present invention to its fullest extent. The following preferred specific embodiments are, therefore, to be construed as merely illustrative, and not limitative of the remainder of the disclosure in any way whatsoever.

Examples 1-14 illustrate compounds wherein X is N rather than O or C(R¹⁷). As shown in Examples 13 and 14, such compounds are effective retroviral protease inhibitors.

All reagents were used as received without purification. All proton and carbon NMR spectra were obtained on either a Varian VXR-300 or VXR-400 nuclear magnetic resonance spectrometer.

### Example 1

### Preparation of [1S-[1R*(R*), 2S*]]-N¹[3-[[[(1,1-dimethylethyl)amino]carbonyl](2-methylpropyl)amino]-2-hydroxy-1-(phenylmethyl)propyl]-2-[(2-quinolinylcarbonyl)amino]-butanediamide

### Part A:

To a solution of 75.0g (0.226 mol) of N-benzyloxycarbonyl-L-phenylalanine chloromethyl ketone in a mixture of 807 mL of methanol and 807 mL of tetrahydrofuran at -2°C, was added 13.17g (0.348 mol, 1.54 equiv.) of solid sodium borohydride over one hundred minutes. The solvents were removed under reduced pressure at 40°C and the residue dissolved in ethyl acetate (approx. 1L). The solution was washed sequentially with 1M potassium hydrogen sulfate, saturated sodium bicarbonate and then saturated sodium chloride solutions. After drying over anhydrous magnesium sulfate and filtering, the solution was removed under reduced pressure. To the resulting oil was added hexane (approx. 1L) and the mixture warmed to 60°C with swirling. After cooling to room temperature, the solids were collected and washed with 2L of hexane. The resulting solid was recrystallized from hot ethyl acetate and hexane to afford 32.3g (43% yield) of N-benzyloxycarbonyl-3 (S)-amino-1-chloro-4-phenyl-2(S)-butanol, mp 150-151°C and M+Li⁺ = 340.

### Part B:

To a solution of 6.52g (0.116 mol, 1.2 equiv.) of potassium hydroxide in 968 mL of absolute ethanol at room temperature, was added 32.3g (0.097 mol) of N-CBZ-3(S)-amino-1-chloro-4-phenyl-2(S)-butanol. After stirring for fifteen minutes, the solvent was removed under reduced pressure and the solids dissolved in methylene chloride. After washing with water, drying over magnesium sulfate, filtering and stripping, one obtains 27.9g of a white solid. Recrystallization from hot ethyl acetate and hexane afforded 22.3g (77% yield) of N-benzyloxycarbonyl-3(S)-amino-1,2(S)-epoxy-4-phenylbutane, mp 102-103°C and MH⁺ 298.

### Part C:

A solution of N-benzyloxycarbonyl 3(S)-amino-1,2-(S)-epoxy-4-phenylbutane (1.00g, 3.36 mmol) and isobutylamine (4.90g, 67.2 mmol, 20 equiv.) in 10 mL of isopropyl alcohol was heated to reflux for 1.5 hours. The solution was cooled to room temperature, concentrated *in vacuo* and then poured into 100 mL of stirring hexane whereupon the product crystallized from solution. The product was isolated by filtration and air dried to give 1.18g, 95% of N=[[3(S)-phenylmethylcarbamoyl)amino-2(R)-hydroxy-4-phenylbutyl]N-[(2-methylpropyl)]amine mp 108.0-109.5°C, MH⁺ m/z = 371.

### Part D:

A solution of [2(R), 3(S)]-N-[[3-(phenylmethylcarbamoyl)amino]-2-hydroxy-4-phenylbutyl]N-[(2-methylpropyl)]amine in 10 ml of tetrahydrofuran was treated with *tert*-butylisocyanate (267 mg, 2.70 mmol) at room temperature for 5 minutes. The solvent was removed *in vacuo* and replaced with ethyl acetate. The ethyl acetate solution was washed with 5% citric acid, water, and brine, dried over anhydrous MgSO₄, filtered and concentrated *in vacuo* to give 1.19g, 97% of [2(R), 3(S)]-N-[[3-(phenylmethylcarbamoyl)amino]-2-hydroxy-4-phenyl]-1-[(2-methylpropyl)]amino-2-(1,1-dimethyl)amino]carbonyl]butane, MH⁺ m/z - 470.

### Part E:

A solution of (1.00g, 2.21 mmol) [2(R), 3(S)]-N-[[3-(phenylmethylcarbamoyl)amino]-2-hydroxy-4-phenyl]-1-[(2-methylpropyl)]amino-1-(1,1-dimethylethyl)amino]carbonyl]butane in 20 mL of methanol was hydrogenated over 10% palladium-on-carbon for 4 hours to give [2(R), 3(S)]-N-[[3-amino]-2-hydroxy-4-phenyl]-1-[(2-methylpropyl)amino-1-(1,1-dimethylethyl)amino]carbonyl]butane 720 mg, 97%.

### Part F:

A solution of *N*-Cbz-L-asparagine (602mg, 2.26 mmol) and *N*-hydroxybenzotriazole (493 mg, 3.22 mmol) in 2mL of dimethylformamide was cooled to 0°C and treated with EDC (473 mg, 2.47 mmol). The solution was allowed to stir at 0°C for 20 minutes and then treated with [2(R), 3(S)]-N-[[3-amino]-2-hydroxy-4-phenyl]-1-[(2-methylpropyl)]amino-1-(1,1-dimethylethyl)amino]carbonyl]butane (720 mg, 2.15 mmol) in 1mL of dimethylformamide. The solution was allowed to warm to room temperature and held at this temperature for 7 hours. The reaction mixture was then poured into 100 mL of 60% saturated aqueous sodium bicarbonate whereupon a white precipitate formed that was isolated by filtration. The filter cake was washed with water, 5% aqueous citric acid, water and then dried in *vacuo* to give 1.04g, 83% of [1S-[1R*(R*), 2S*]]-N¹[3-[[[(1,1-dimethylethyl)amino]carbonyl](2-methylpropyl)amino], mp. 164.0-166.5°C, MH⁺ m/z = 584.

### Part G.

A solution of [1S-[1R*(R*), 2S*]]-N¹[3-[[[(1,1-dimethylethyl)amino]carbonyl](2-methylpropyl)amino]-2-hydroxy-1-(phenylmethyl)propyl]-2-[(phenylmethylcarbamoyl)amino]-butanediamide (1.00g, 1.72 mmol) in 10 mL of methanol was hydrogenated over 10% palladium-on-carbon for 4 hours to give [1S-[1R*(R*), 2S*]]-N¹[3-[[[(1,1-dimethylethyl) amino]carbonyl](2-methylpropyl)amino]-2-hydroxy-1-(phenylmethyl)propyl]-2-amino]-butanediamide, 784mg, 99%.

### Part H:

A mixture of [1S-[1R*(R*), 2S*]]-N¹[3-[[[(1,1-dimethylethyl)amino]carbonyl](2-methylpropyl)amino]-2-hydroxy-1-(phenylmethyl)propyl]-2-amino]-butanediamide, (784 mg, 1.70 mmol), 2-quinoline carboxylic acid N-hydroxysuccinimide ester (459 mg, 1.70 mmol), N-methylmorpholine (343 mg, 3.40 mmol) in 5 mL of dichloromethane was stirred at room temperature for 15 minutes. The solvent was removed *in vacuo* and replaced with ethyl acetate and the solution washed with 5% aqueous citric acid, saturated aqueous sodium bicarbonate, brine, dried over anhydrous MgSO₄, filtered and concentrated in *vacuo.* The crude product was recrystallized from acetone/hexane to give 790 mg, 77% of [1S-[1R*(R*), 2S*]]-N¹[3-[[[(1,1-dimethylethyl)amino]carbonyl](2-methylpropyl)amino]-2-hydroxy-1-(phenylmethyl)propyl]-2-[(2-quinolinylcarbonyl)amino]-butanediamide, mp 107.0-109.8°C, MH⁺ = 605.

### Example 2

The procedure described in Example 1, part C-H, was used to prepare [1S-[1R*(R*), 2S*]]-N¹[3-[[[(1,1-dimethylethyl)amino]carbonyl](3-methylbutyl)amino]-2-hydroxy-1-(phenylmethyl)propyl]-2-[(2-quinolinylcarbonyl)amino]-butanediamide.
a) From the reaction of 1.06g (3.56mmol) of N-benzyloxycarbonyl 3(S)-amino-1,2-(S)-epoxy-4-phenylbutane and 6.25g (71.7mmol) of isoamylamine, one obtains 1.27g (92%) of [2(R), 3(S)]-N-[[3-(phenylmethylcarbamoyl)amino]-2-hydroxy-4-phenylbutyl]N-[(3-methylbutyl)]amine, mp 130-132C and MH* 385. This amine (400mg, 1.04mmol) was then reacted with tert-butylisocyanate (110mg, 1.11mmol) to afford 500mg (100%) of [2(R), 3(S)]-N-[[3-(phenylmethylcarbamoyl)amino]-2-hydroxy-4-phenyl]-1-[(3-methylbutyl)]amino-1-(1,1-dimethylethy)amino]carbonyl]butane; as an oil, MH⁺ 484.
b) The CBZ protected compound (530mg, 1.10mmol) was then deprotected by hydrogenation over 10% palladium-on-carbon and the resulting free amine coupled with N-CBZ-L-asparagine (377mg, 1.42mmol) in the presence of N-hydroxybenzotriazole (290mg, 2.15mmol) and EDC (300mg, 1.56mmol) to yield 430mg (53%) of [1S-[1R*(R*), 2S*]]-N¹[3-[[[(1,1-dimethylethyl) amino]carbonyl](3-methylbutyl)amino]-2-hydroxy-1-(phenylmethyl)propyl]-2-[(phenylmethylcarbamoyl)amino]-butanediamide, mp 148-151 C (dec) and MH⁺ 598. This compound (370mg, 0.619mmol) was then deprotected by hydrogenation over 10% palladium-on-carbon and the resulting free amine coupled with 2-quinolinecarboxylic acid N-hydroxy-succinimide ester (193mg, 0.714mmol), in the presence of N-methylmorpholine, to afford 310mg (70%) of pure [1S-[1R*(R*), 2S*]]-N¹[3-[[[(1,1-dimethylethyl)amino]carbonyl](3-methylbutyl)amino]-2-hydroxy-1-(phenylmethyl)propyl]-2-[(2-quinolinylcarbonyl)amino]-butanediamide; mp 93.5-95.5C and MH⁺ 619.

### Example 3

The procedure described in Example 1, part C-H, was used to prepare [1S-[1R*(R*), 2S*]]-N¹[3-[[[(1,1-dimethylethyl)amino]carbonyl]2-napthylmethyl)amino]-2-hydroxy-1-(phenylmethyl)propyl]-2-[(2-quinolinylcarbonyl)amio]-butanediamide.
a) From the reaction of 1.80g (6.05mmol) of N-benzyloxycarbonyl 3(S)-amino-1,2-(S)-epoxy-4-phenylbutane and 1.15g (7.31mmol) of 2-(aminomethyl)naphthalene, one obtains 2.11g (77%) of [2(R), 3(S)]-N-[[3-(phenylmethylcarbamoyl)amino]-2-hydroxy-4-phenylbutyl]N-[(2-napthylmethyl)]amine, MH⁺ 455. This amine (366.8mg, 0.807mmol) was then reacted with tert-butylisocyanate (66.4mg, 0.67mmol) to afford 350.0mg (94%) of [2(R), 3(S)]-N-[[3-(phenylmethylcarbamoyl)amino]-2-hydroxy-4-phenyl]-1-[(2-napthylmethyl)]amino-1-(1,1-dimethylethyl)amino]carbonyl]butane; as an oil, MH⁺ 554.
b) The CBZ protected compound (330mg, 0.596mmol) was then deprotected by hydrogenation over 10% palladium-on-carbon and the resulting free amine coupled with N-CBZ-L-asparagine (165.1mg, 0.62mmol) in the presence of N-hydroxybenzotriazole (142.3mg, 0.93mmol) and EDC (130.7mg, 0.68mmol) to yield 161.7mg (41%) of [1S-[1R*(R*), 2S*]]-N¹[3-[[[(1,1-dimethylethyl)amino]carbonyl] (2-napthylmethyl)amino]-2-hydroxy-1-(phenylmethyl)propyl]-2-[(phenylmethylcarbamoyl)amino]-butanediamide; mp 151-152 C (dec) and MH⁺ 668. This compound (91.0mg, 0.136mmol) was then deprotected by hydrogenation over 10% palladium-on-carbon and the resulting free amine coupled with 2-quinolinecarboxylic acid N-hydroxysuccinimide ester (36.8mg, 0.136mmol), in the presence of N-methylmorpholine, to afford 65.8mg (70%) of pure [1S-[1R*(R*), 2S*]]- N¹[3-[[[(1,1-dimethylethyl)amino]carbonyl](2-napthylmethyl)amino]-2-hydroxy-1-(phenylmethyl)propyl]-2-[(2-quinolinylcarbonyl)amino]-butanediamide; mp 119-120C and MH⁺ 689.

### Example 4

The procedure described in Example 1, part C-H, was used to prepare [1S-[1R*(R*), 2S*]]-N¹[3-[[[(1,1-dimethylethyl)amino]carbonyl](2-phenylethyl)amino]-2-hydroxy-1-(phenylmethyl)propyl]-2-[(2-quinolinylcarbonyl)amino]-butanediamide.
a) From the reaction of 1.00g (3.36mmol) of N-benzyloxycarbonyl 3(S)-amino-1,2-(S)-epoxy-4-phenylbutane and 8.19g (67.0mmol) of 2-phenethyl amine, one obtains 1.10g (79%) of [2(R), 3(S)]-N-[[3-(phenylmethylcarbamoyl)amino]-2-hydroxy-4-phenylbutyl]N-[(2-phenylethyl)]amine, mp 137-138 C and MH⁺ 419. This amine (750mg, 1.79mmol) was then reacted with tert-butylisocyanate (178mg, 1.79mmol) to afford 897mg ■(97%) of [2(R), 3(S)]-N-[[3-(phenylmethylcarbamoyl)amino]-2-hydroxy-4-phenyl]-1-[(2-phenylethyl)]amino-1-(1,1-dimethylethyl)amino]carbonyl]butane; as an oil, MH⁺ 518.
b) The CBZ protected compound (897mg, 1.73mmol) was then deprotected by hydrogenation over 10% palladium-on-carbon and the resulting free amine coupled with N-CBZ-L-asparagine (620.7mg, 2.33mmol) in the presence of N-hydroxybenzotriazole (509.5mg, 3.33mmol) and EDC (488.Omg, 2.55mmol) to yield 1.00g (92%) of [1S-[1R*(R*), 2S*]]- N¹[3[[[(1,1-dimethylethyl)amino]carbonyl](2-phenylethyl)amino]-2-hydroxy-1-(phenylmethyl)propyl]-2-[(phenylmethylcarbamoyl)amino]-butanediamide; mp 145 (dec) and MH⁺ 632. This compound (860mg, 1.36mmol) was then deprotected by hydrogenation over 10% palladium-on-carbon and the resulting free amine coupled with 2-quinolinecarboxylic acid N-hydroxysuccinimide ester (338mg, 1.25mmol), in the presence of N-methylmorpholine, to afford 450.4mg (55%) of pure [1S-[1R*(R*), 2S*]]-N¹[3[[[(1,1-dimethylethyl)amino]carbonyl](2-phenylethyl)amino]-2-hydroxy-1-(phenylmethyl)propyl]-2-[(2-quinolinylcarbonyl)amino]-butanediamide; mp 139-140°C and MH⁺ 653.

### Example 5

The procedure described in Example 1, part C-H, was used to prepare [1S-[1R*(R*), 2S*]]-N¹[3-[[[(1,1-dimethylethyl)amino]carbonyl](2,2-dimethylpropyl)amino]-2-hydroxy-1-(phenylmethyl)propyl]-2-[(2-quinolinylcarbonyl)amino]-butanediamide.
a) From the reaction of 1.00g (3.36mmol) of N-benzyloxycarbonyl 3(S)-amino-1,2-(S)-epoxy-4-phenylbutane and 7.9mL (approx. 67mmol) of neopentyl amine, one obtains 0.69g (49%) of [2(R), 3(S)]-N-[[3-(phenylmethylcarbamoyl)amino]-2-hydroxy-4-phenylbutyl]N-[(2,2-dimethylpropyl)]amine, MH⁺ 385. This amine (686mg, 1.78mmol) was then reacted with tert-butylisocyanate (180mg, 1.78mmol) to afford 860mg (100%) of [2(R), 3(S)]-N-[[3-(phenylmethylcarbamoyl)amino]-2-hydroxy-4-phenyl]-1-[(2,2-dimethylpropyl)]amino-1-(1,1-dimethylethyl)amino]carbonyl]butane; MH⁺ 484.
b) The CBZ protected compound (860mg, 1.78mmol) was then deprotected by hydrogenation over 10% palladium-on-carbon and the resulting free amine coupled with N-CBZ-L-asparagine (471mg, 1.77mmol) in the presence of N-hydroxybenzotriazole (406mg, 2.66mmol) and EDC (374mg, 1.95mmol) to yield 326mg (34%) of [1S-[R*(R*), 2S*]]-N¹[3-[[[(1,1-dimethylethyl)amino]carbonyl](2,2-dimethylpropyl)amino]-2-hydroxy-1-(phenylmethyl)propyl]-2-[(phenylmethylcarbamoyl)amino]-butanediamide; mp 177-178C and MH⁺ 598. This compound (245mg, 0.41mmol) was then deprotected by hydrogenation over 10% palladium-on-carbon and the resulting free amine coupled with 2-quinolinecarboxylic acid N-hydroxy-succinimide ester (111mg, 0.41mmol), in the presence of N-methylmorpholine, to afford 150mg (59%) of pure [1S-[R*(R*), 2S*]]-N¹[3-[[[(1,1-dimethylethyl)amino]carbonyl](2,2-dimethylpropyl)amino]-2-hydroxy-1-(phenylmethyl)propyl]-2-[(2-quinolinylcarbonyl)amino]-butanediamide; mp 115-117C and MH⁺ 619.

### Example 6

The procedure described in Example 1, part C-H, was used to prepare [1S-[R*(R*), 2S*]]-N¹[3-[[[(1,1-dimethylethyl)amino]carbonyl] (4-methoxyphenylmethyl)amino]-2-hydroxy-1-(phenylmethyl)propyl]-2-[(2-quinolinylcarbonyl)amino]-butanediamide;
a) From the reaction of 1.00g (3.36mmol) of N-benzyloxycarbonyl 3(S)-amino-1,2-(S)-epoxy-4-phenylbutane and 9.2g (67mmol) of 4-methoxybenzyl amine, one obtains 1.12g (76%) of [2(R), 3(S)]-N-[[3-(phenylmethylcarbamoyl)amino]-2-hydroxy-4-phenylbutyl]N-[(4-methoxyphenylmethyl)]amine, MH⁺ 435. This amine (1.12g, 2.58mmol) was then reacted with tert-butylisocyanate (260mg, 2.58mmol) to afford 1.35g (98%) of [2(R), 3(S)]-N-[[3-(phenylmethylcarbamoyl)amino]-2-hydroxy-4-phenyl]-1-[(4-methoxyphenylmethyl)]amino-1-(1,1-dimethylethyl)amino]carbonyl]butane; MH⁺ 534.
b) The CBZ protected compound (1.35g, 2.53mmol) was then deprotected by hydrogenation over 10% palladium-on-carbon and the resulting free amine coupled with N-CBZ-L-asparagine (684mg, 2.57mmol) in the presence of N-hydroxybenzotriazole (590mg, 3.85mmol) and EDC (543mg, 2.83mmol) to yield 442mg (29%) of [1S-[1R*(R*), 2S*]]-N¹[3-[[[(1,1-dimethylethyl)amino]carbonyl](4-methoxyphenylmethyl)amino]-2-hydroxy-1-(phenylmethyl)propyl]-2-[phenylmethylcarbamoyl)amino]-butanediamide; mp 175C (dec) and MH⁺ 648. This compound (345mg, 0.53mmol) was then deprotected by hydrogenation over 10% palladium-on-carbon and the resulting free amine coupled with 2-quinolinecarboxylic acid N-hydroxy-succinimide ester (118mg, 0.44mmol), in the presence of N-methylmorpholine, to afford 108mg (31%) of pure [1S-[1R*(R*), 2S*]]-N¹[3-[[[(1,1-dimethylethyl)amino]carbonyl](4-methoxyphenylmethyl)amino]-2-hydroxy-1-(phenylmethyl)propyl]-2-[(2-quinolinylcarbonyl)amino]-butanediamide; mp 220C (dec) and MLi⁺ 675.

### Example 7

The procedure described in Example 1, part C-H, was used to prepare [1S-[1R*(R*), 2S*]]-N¹[3-[[[(1,1-dimethylethyl)amino]carbonyl](n-butyl)amino]-2-hydroxy-1-(phenylmethyl)propyl]-2-[(2-quinolinylcarbonyl)amino]-butanediamide.
a) From the reaction of 1.48g (5.0mmol) of N-benzyloxycarbonyl 3(S)-amino-1,2-(S)-epoxy-4-phenylbutane and 7.314g (100.0mmol) of n-butyl amine, one obtains 1.50g (80%) of [2(R), 3(S)]-N-[[3-(phenylmethylcarbamoyl)amino]-2-hydroxy-4-phenylbutyl]N-[n-butyl)]amine. This amine (1.48g, 4.0mmol) was then reacted with tert-butylisocyanate (396mg, 4.0mmol) to afford 1.87g (100%) of [2(R), 3(S)]-N-[[3-(phenylmethylcarbamoyl)amino]-2-hydroxy-4-phenyl]-1-[(n-butyl)]amino-1-(1,1-dimethylethyl)amino]carbonyl] butane as an oil.
b) The CBZ protected compound (1.87g, 4.0mmol) was then deprotected by hydrogenation over 10% palladium-on-carbon and the resulting free amine coupled with N-CBZ-L-asparagine (1.05g, 3.96mmol) in the presence of N-hydroxybenzotriazole (535mg, 7.9mmol) and EDC (759mg, 3.96mmol) to yield 1.75g (76%) of [1S-[1R*(R*), 2S*]]-N¹[3-[[[(1,1-dimethylethyl)amino]carbonyl] (n-butyl)amino]-2-hydroxy-1-(phenylmethyl)propyl]-2-[(phenylmethylcarbamoyl)amino]-butanediamide; mp 166-167C and MH⁺ 584.

### Example 8

The procedure described in Example 1, part C-H, was used to prepare [1S-[1R*(R*), 2S*]]-N¹[3-[[[(1,1-dimethylethyl)amino]carbonyl] (phenylmethyl)amino]-2-hydroxy-1-(phenylmethyl)propyl]-2-[(2-quinolinylcarbonyl)amino]-butanediamide.
a) From the reaction of 1.48g (5.0mmol) of N-benzyloxycarbonyl 3(S)-amino-1,2-(S)-epoxy-4-phenylbutane and 10.68g (100.0mmol) of benzyl amine, one obtains 1.88g (95%) of [2(R), 3(S)]-N-[[3-(phenylmethylcarbamoyl)amino]-2-hydroxy-4-phenylbutyl]N-[(phenylmethyl)]amine. This amine (1.88g, 4.65mmol) was then reacted with tert-butylisocyanate (460.0mg, 4.6mmol) to afford 2.24g (96%) of [2(R), 3(S)]-N-[[3-(phenylmethylcarbamoyl)amino]-2-hydroxy-4-phenyl]-1-[(phenylmethyl)]amino-1-(1,1-dimethylethyl)amino]carbonyl] butane.
b) The CBZ protected compound (2.22g, 4.4mmol) was then deprotected by hydrogenation over 10% palladium-on-carbon and the resulting free amine coupled with N-CBZ-L-asparagine (1.17g, 4.4mmol) in the presence of N-hydroxybenzotriazole (1.19g, 8.8mmol) and EDC (843mg, 4.4mmol) to yield 2.11g (78%) of [1S-[1R*(R*), 2S*]]-N¹[3-[[[(1,1-dimethylethyl)amino]carbonyl](phenylmethyl)amino]-2-hydroxy-1-(phenylmethyl)propyl]-2-[(phenylmethylcarbamoyl)amino]-butanediamide; mp 156-158C and MH⁺ 618. This compound (1.0g, 1.62mmol) was then deprotected by hydrogenation over 10% palladium-on-carbon and the resulting free amine coupled with 2-quinolinecarboxylic acid N-hydroxysuccinimide ester (437mg, 1.62mmol), in the presence of N-methylmorpholine, to afford 640mg (62%) of pure [1S-[1R*(R*), 2S*]]-N¹[3-[[[(1,1-dimethylethyl)amino]carbonyl](phenylmethyl)amino]-2-hydroxy-1-(phenylmethyl)propyl]-2-[(2-quinolinylcarbonyl)amino]-butanediamide; mp 110.5-112.5C and MH⁺ 639.

### Example 9

This example illustrates preparation of compounds of Formula IV wherein t is 1.

### 4-N-benzyl itaconamide.

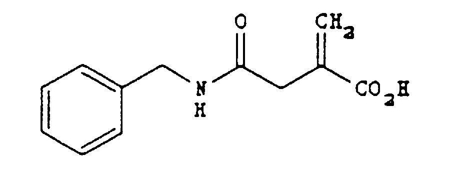

A 500 mL three necked round bottomed flask equipped with a dropping funnel, mechanical stirrer, nitrogen inlet and reflux condenser was charged with itaconic anhydride (33.6g, 0.3 mol) and 150 mL of toluene. This solution was added a solution of benzylamine (32.1g, 0.3 mol) in 50 mL of toluene dropwise over 30 m at room temperature. The solution was stirred at this temperature an additional 3h and then the solid product isolated by filtration on a Büchner funnel. The crude product, 64.6g 98%, was recrystallized from 300 mL of isopropyl alcohol to give after two crops 52.1g, 79% of pure product, mp 149-150 °C

### 2(R)-Methyl 4-N-benzyl succinamide.

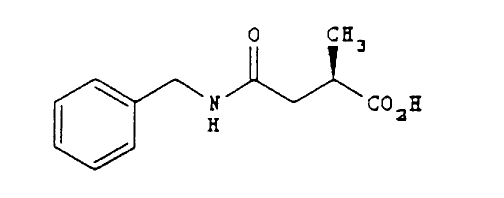

A large Fisher-Porter bottle was charged with the acid from the above reaction (10.95g, 0.05 mol), rhodium (R,R)-DiPAMP (220mg, 0.291 mmol) and 125 mL of degassed methanol. The solution was then hydrogenated at 40 psig for 16h at room temperature. After the hydrogen uptake ceased, the vessel was opened and the solution concentrated *in vacuo* to give a yellow solid, 11.05g, 100%. The product was then taken up in absolute ethanol and allowed to stand whereupon crystals of the desired product formed, 7.98g, 72%, mp 127-129 °C [a]_{D} @ 25 °C=+14.9° (c=1.332, EtOH), ¹H nmr (CDCl₃) 300MHz 7.30(m,5H), 6.80(brs, 1H), 4.41(d, J=5.8Hz, 2H), 2.94(m, 1H), 2.62(dd, J=8.1, 14.9Hz, 1H), 2.33(dd, J=5.5, 14.9Hz, 1H), 1.23(d, J=7.2Hz, 3H).

### 4-N(4-methoxybenzyl)itaconamide.

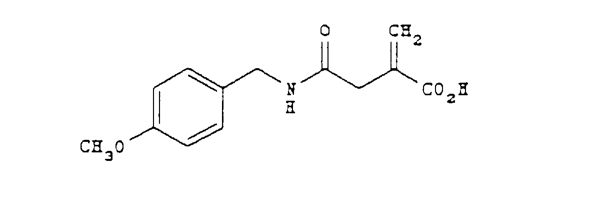

A 500 mL three necked round bottomed flask equipped with a dropping funnel, mechanical stirrer, nitrogen inlet and reflux condenser was charged with itaconic anhydride (44.8g, 0.4 mol) and 150 mL of toluene. This solution was added a solution of 4-methoxybenzylamine (54.8g, 0.4 mol) in 50 mL of toluene dropwise over 30 m at room temperature. The solution was stirred at this temperature an additional 2h and then the solid product isolated by filtration on a Büchner funnel. The crude product was recrystallized from ethyl acetate/ethanol to give after two crops 64.8g, 65% of pure product, mp 132-134 °C, ¹H nmr (CDCl₃) 300MHz 7.09(d, J=9.1Hz, 2H), 6.90(brt, J=5.9Hz, 1H), 6.74(d, J=9.1Hz, 2H), 6.22(s, 1H), 5.69(s, 1H), 4.24(d, J=5.9Hz, 2H), 3.69(s, 3H), 3.15(s, 2H). ¹³C nmr (CDCl₃) 170.52, 169.29, 159.24, 135.61, 131.08, 129.37, 128.97, 114.36, 55.72, 43.37, 40.58.

### 2(R)-Methyl 4-N(4-methoxybenzyl)succinamide.

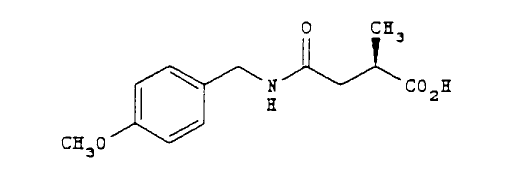

A large Fisher-Porter bottle was charged with the acid from the above reaction (5.00 g, 0.02 mol), rhodium (R,R)-DiPAMP (110 mg, 0.146 mmol) and 50 mL of degassed methanol. The starting acid was not completely soluble initially, but as the reaction progressed the solution became homogeneous. The solution was then hydrogenated at 40 psig for 16h at room temperature. After the hydrogen uptake ceased, the vessel was opened and the solution concentrated in *vacuo* to give a yellow solid. The crude product was then taken up in ethyl acetate and washed three times with sat. aq. NaHCO₃ solution. The combined aqueous extracts were acidified to pH=1 with 3 N HCl and then extracted three times with ethyl acetate. The combined ethyl acetate extracts were washed with brine, dried over anhyd. MgSO₄, filtered and concentrated to give the expected product as a white solid, 4.81g, 95%. This material was recrystallized from a mixture of methyl ethyl ketone/hexane to give 3.80g, 75% of pure product, [a]_{D} @ 25 °C=+11.6° (c=1.572, MeOH). ¹H nmr (CDCl₃) 300MHz 11.9(brs, 1H), 7.18(d, J=9.2Hz, 2H), 6.82(d, J=9.2Hz, 2H), 6.68(brt, J=5.6Hz, 1H), 4.33(d, J=5.6Hz, 2H), 3.77(s, 3H), 2.92(ddq, J=7.9, 5.4, 7.3Hz, 1H), 2.60(dd, J=5.4, 15.0Hz, 1H), 2.30(dd, J=7.9, 15.0Hz, 1H),1.22(d, J=7.3Hz, 3H).

### Butanediamide, N'-[3-[[[(1,1-dimethylethyl)amino]carbonyl] (3-methylbutyl)amino]-2-hydroxy-1-(phenylmethyl)propyl)-N-4-methoxyphenylmethyl-2-methyl, [1S-[1R*(2R*),2S*]]-

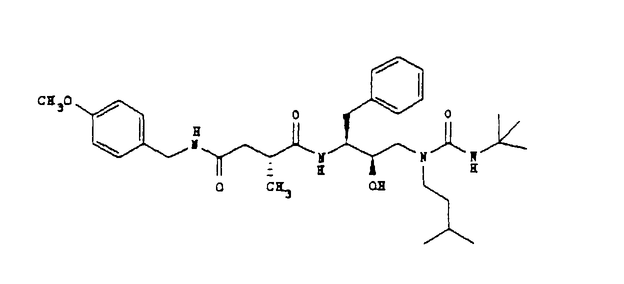

A 50 mL round bottomed flask was charged with 2(R)-methyl 4-N(4-methoxybenzyl)succinamide (588 mg, 2.35 mmol), N-hydroxybenzotriazole (511 mg, 3.34 mmol) and 6 mL of DMF. The solution was cooled to 0° C and treated with 1-(3-dimethylaminopropyl)-3-ethylcarbodiimide hydrochloride (502 mg, 2.62 mmol) for 20 m. A solution of (2R,3S)-3-amino-1-(3-methylbutyl)-1-[(1,1-dimethylethyl)amino]carbonyl)-4-phenyl-2-butanol (782 mg, 2.24 mmol) in 2 mL of DMF was added and the solution stirred at room temperature for a period of 24 h. The solution was concentrated *in vacuo* and poured into 50 mL of 50% sat. aq. NaHCO₃, the aqueous phase was extracted with CH₂Cl₂. The organic phase was washed with 5% citric acid, NaHCO₃, brine, dried over anhyd. MgSO₄, filtered and concentrated to give an oil that was purified by radial chromatography on SiO₂ eluting with hexane/ethyl acetate to give 790 mg, 59% of pure product as a white foam.

### Butanediamide, N'-[3-[[[(1,1-dimethylethyl)amino]carbonyl] (3-methylbutyl)amino]-2-hydroxy-1-(phenylmethyl)propyl]-N-phenylmethyl-2-methyl, [1S-[1R*(2R*),2S*]]-

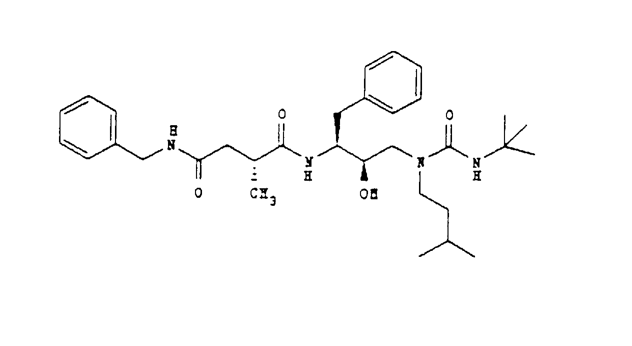

A 50 mL round bottomed flask was charged with 2(R)-methyl 4-N-(benzyl) succinamide (243 mg, 1.1 mmol), N-hydroxybenzotriazole (213 mg, 1.39 mmol) and 3 mL of DMF. The solution was cooled to 0° C and treated with 1-(3-dimethylaminopropyl)-3-ethylcarbodiimide hydrochloride (228 mg, 1.17 mmol) for 20 m. A solution of (2R,3S)-3-amino-1-(3-methylbutyl)-1-[(1,1-dimethylethyl)amino]carbonyl)-4-phenyl-2-butanol (327 mg, 0.95 mmol) in 2 mL of DMF was added and the solution stirred at room temperature for a period of 24 h. The solution was concentrated *in vacuo* and poured into 50 mL of 50% sat. aq. NaHCO₃, the aqueous phase was extracted with CH₂Cl₂. The organic phase was washed with 5% citric acid, NaHCO₃, brine, dried over anhyd. MgSO₄, filtered and concentrated to give an oil that was purified by flash chromatography on SiO₂ eluting with hexane/ethyl acetate to give 370 mg, 70% of pure product as a white foam.

### Example 10

Following the procedure generally as set forth in Example 28, the compounds shown in Table 14 were prepared.

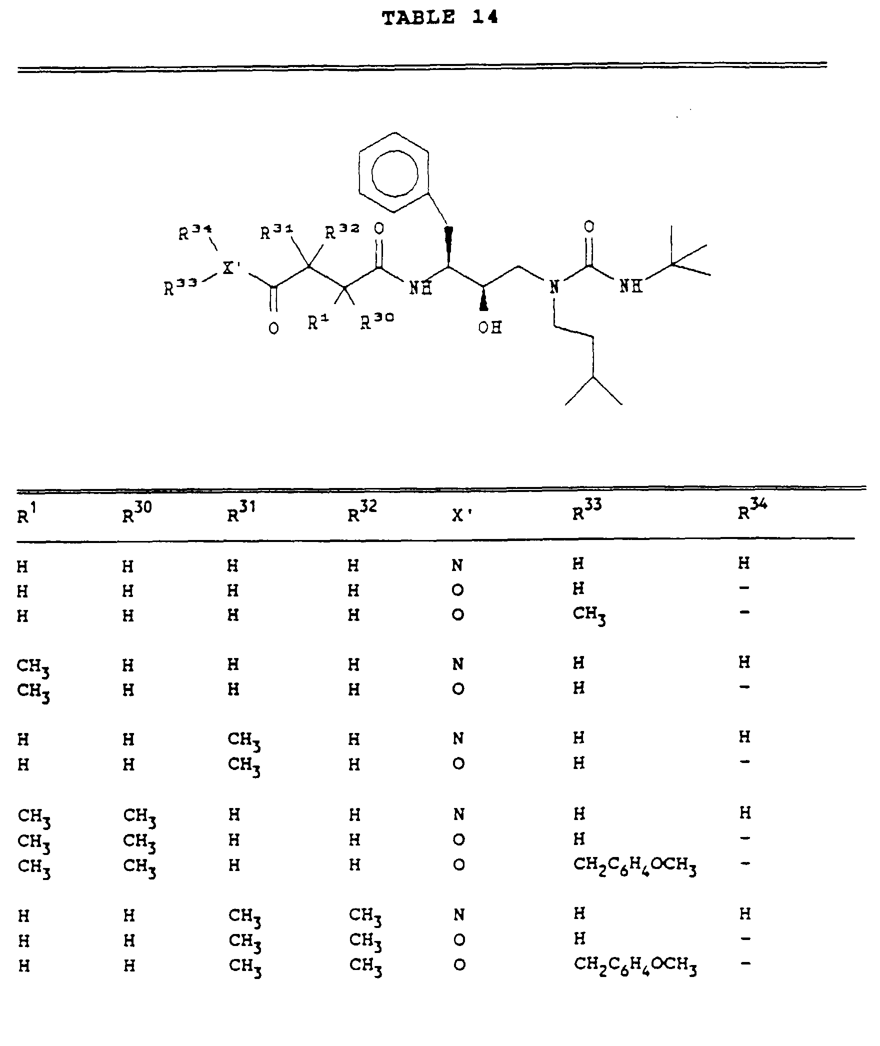

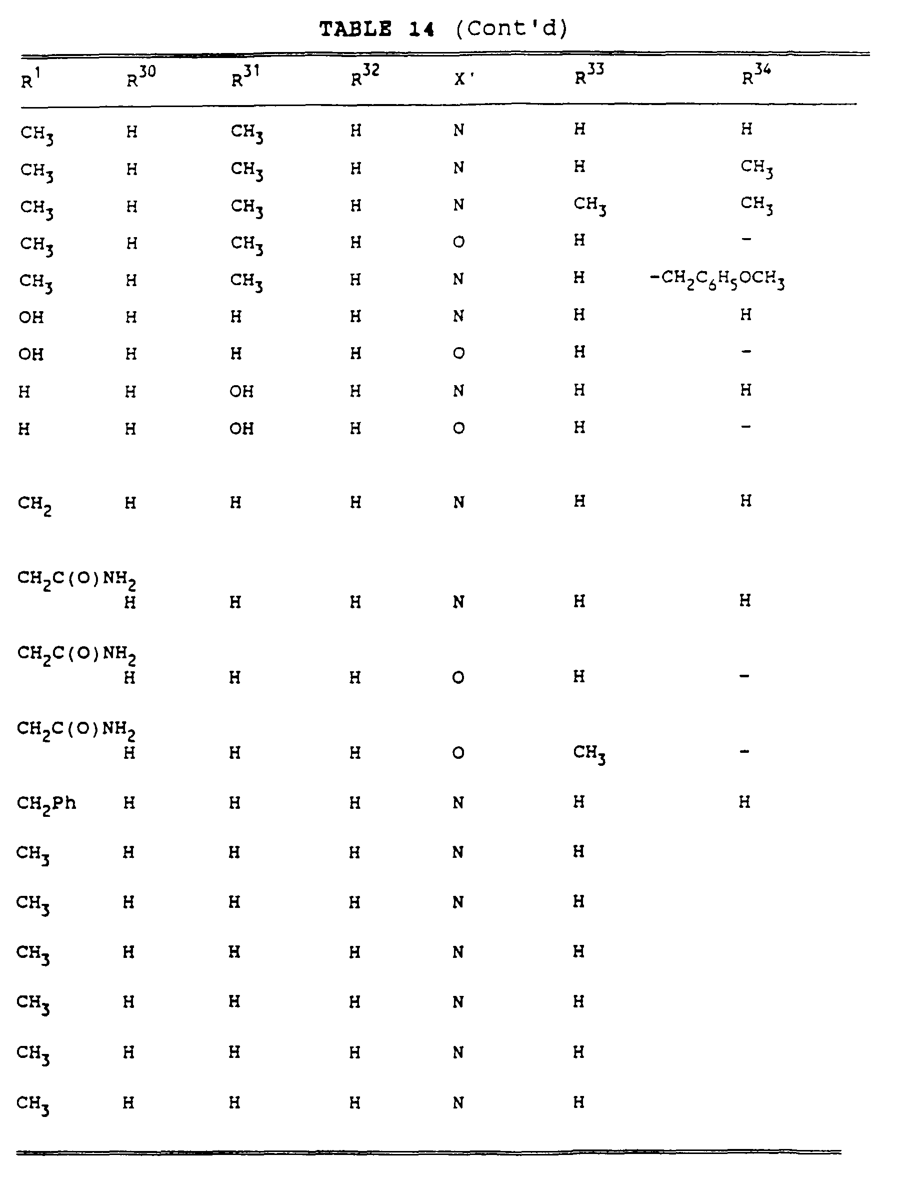

### Example 11

Following the procedure generally as set forth in Example 9, the compounds shown in Table 15 were prepared.

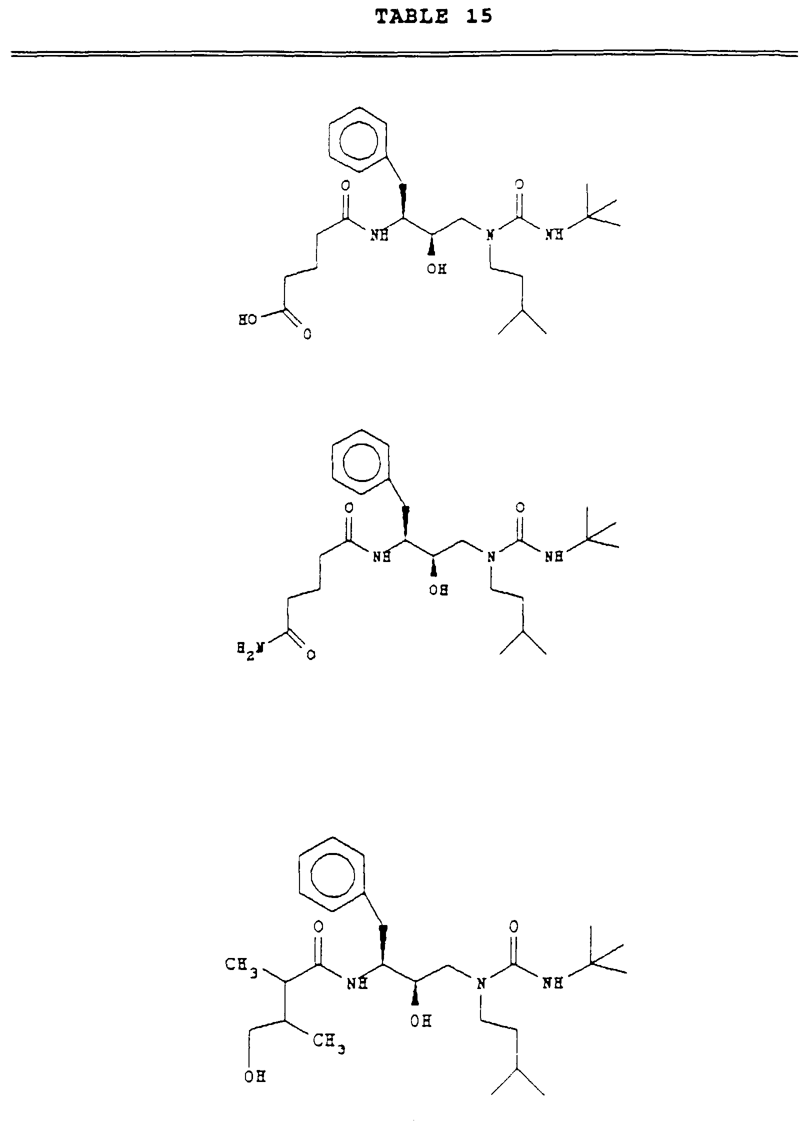

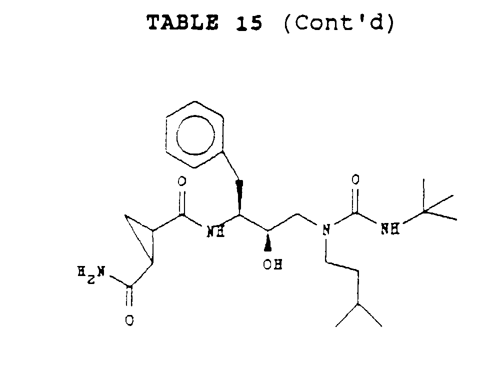

### Example 12

This example illustrates the procedure utilized to prepare compounds wherein the stereochemistry about the hydroxyl group is (S).

### Part A:

A solution of 3(S)-(1,1-dimethylethoxycarbonyl)amino-1,2-(R)-epoxy-4-phenylbutane (1.00g, 3.80 mmol) and isobutylamine (5.55g, 76 mmol, 20 equiv.) in 10 mL of isopropyl alcohol was warmed to 60°C for 1 hour. The solution was cooled to room temperature and concentrated in *vacuo* and the residue recrystallized from hexane/methylene chloride to give 0.93g, 73% of [2(S), 3(S)]-N-[[[3-[(1,1-dimethylethyl)carbamoyl]amino]]-2-hydroxy-4-phenylbutyl]N-[(3-methylbutyl)]amine, mp 91.3 - 93.0°C.

### Part B:

The product from Part A (46.3mg, 0.14 mmol) was dissolved in a mixture of 5 mL of tetrahydrofuran and 2 mL of methylene chloride and treated with tert-butylisocyanate (136.4mg, 1.376 mmol) *via* syringe. The solution was stirred at room temperature for 0.5 hour and then the solvent was removed *in vacuo.* The product, TLC on SiO₂, 1:1 hexane: Ethyl acetate had Rf = 0.74 and was used directly in the next step without further purification.

### Part C:

The crude product from Part B was taken up in 10 mL of 4N hydrochloric acid in dioxane and stirred at room temperature for 0.25 hours. The solvent and excess hydrochloric acid was removed *in vacuo* whereupon the product crystallized. The solid was isolated by filtration washed with acetone and dried *in vacuo* to 3-[[(1,1-dimethylethyl)amino]carbonyl](2-methylpropyl)amino-2(S)-hydroxy-1(S)-(phenylmethyl)propylamine hydrochloride.

### Part D:

A solution of N-Cbz-L-asparagine (225.5mg, 0.847 mmol) and N-hydroxybenzotriazole (182.9mg, 1.21 mmol) was dissolved in 2 mL of dimethylformamide and cooled to 0°C and then treated with EDC (170.2mg, 0.898 mmol) for 10 minutes. This mixture was then treated with 3-[[(1,1-dimethylethyl)amino]carbonyl] (2-methylpropyl)amino-2(S)-hydroxy-1(S)-(phenylmethyl)propylamine hydrochloride. (300.0mg, 0.807 mmol) followed by N-methylmorpholine (90.0mg, 0.888 mmol) via syringe. The solution was stirred at room temperature for 16 hours and then poured into 20 mL of rapidly stirring 60% saturated aqueous sodium bicarbonate solution whereupon a white precipitate formed. The solid was isolated by filtration, washed with saturated aqueous sodium bicarbonate solution, water, 5% aqueous citric acid solution, water and then dried *in vacuo* to give 319mg, 68% of butanediamide, N¹-[3-[[[(1,1-dimethylethyl)amino]carboyl](2-methylpropyl)amino]-2(S)-hydroxy-1(S)-(phenylmethyl)propyl]-2(S)-[(benzyloxycarbonyl)amino] mp 139-141°C, MH⁺ m/z = 584.

### EXAMPLE 13

The compounds of the present invention are effective HIV protease inhibitors. Utilizing an enzyme assay as described below, the compounds set forth in the examples herein disclosed inhibited the HIV enzyme. The preferred compounds of the present invention and their calculated IC₅₀ (inhibiting concentration 50%, i.e., the concentration at which the inhibitor compound reduces enzyme activity by 50%) values are shown in Table 18. The enzyme method is described below. The substrate is 2-aminobenzoyl-Ile-Nle-Phe(p-NO₂)-Gin-ArgNH₂. The positive control is MVT-101 (Miller, M. et al, Science, 246, 1149 (1989)] The assay conditions are as follows: Assay buffer:
20 mM sodium phosphate, pH 6.4
20% glycerol
1 mM EDTA
1 mM DTT
0.1% CHAPS

The above described substrate is dissolved in DMSO, then diluted 10 fold in assay buffer. Final substrate concentration in the assay is 80 µM.

HIV protease is diluted in the assay buffer to a final enzyme concentration of 12.3 nanomolar, based on a molecular weight of 10,780.

The final concentration of DMSO is 14% and the final concentration of glycerol is 18%. The test compound is dissolved in DMSO and diluted in DMSO to lOx the test concentration; 10µl of the enzyme preparation is added, the materials mixed and then the mixture is incubated at ambient temperature for 15 minutes. The enzyme reaction is initiated by the addition of 40µl of substrate. The increase in fluorescence is monitored at 4 time points (0, 8, 16 and 24 minutes) at ambient temperature. Each assay is carried out in duplicate wells.

**TABLE 18**

| Compound | IC₅₀(nanomolar) |
|---|---|
| 1. Butanediamide, N¹-[3-[[[(2,2-dimethyl)propyl]carbonyl] (3-methylbutyl)amino]-2-hydroxy-1-(phenylmethyl)propyl]-2-[(2-quinolinylcarbonyl) amino]-, [1S-[1R*(2R*),2S*]] | 21nM |
| 2. Butanediamide, N¹-[3-[[butylcarbonyl](cyclohexylmethyl)amino]-2-hydroxy-1-(phenylmethyl) propyl]-2-[(phenylmethyloxy)-carbonyl)amino]-, [1S-[1R*(2R*),2S*]] | 696nM |
| 3. Carbamic acid, [3-[[4-amino-1,4-dioxo-2-[(phenylmethyloxy)carbonyl] amino]butyl]amino]-2-hydroxy-4-phenylbutyl](phenylmethyl)-, butyl ester, [2R- [2R*,3S*(S*)]] | 1.6mM |

### Example 14

The effectiveness of the compounds listed in Table 19 were determined in the above-described enzyme assay and in a CEM cell assay.

The HIV inhibition assay method of acutely infected cells is an automated tetrazolium based colorimetric assay essentially that reported by Pauwles et al, J. Virol. Methods 20, 309-321 (1988). Assays were performed in 96-well tissue culture plates. CEM cells, a CD4⁺ cell line, were grown in RPMI-1640 medium (Gibco) supplemented with a 10% fetal calf serum and were then treated with polybrene (2µg/ml). An 80 µl volume of medium containing 1 x 10⁴ cells was dispensed into each well of the tissue culture plate. To each well was added a 100µl volume of test compound dissolved in tissue culture medium (or medium without test compound as a control) to achieve the desired final concentration and the cells were incubated at 37°C for 1 hour. A frozen culture of HIV-1 was diluted in culture medium to a concentration of 5 x 10⁴ TCID₅₀ per ml (TCID₅₀ = the dose of virus that infects 50% of cells in tissue culture), and a 20µL volume of the virus sample (containing 1000 TCID₅₀ of virus) was added to wells containing test compound and to wells containing only medium (infected control cells). Several wells received culture medium without virus (uninfected control cells). Likewise, the intrinsic toxicity of the test compound was determined by adding medium without virus to several wells containing test compound. In summary, the tissue culture plates contained the following experiments:

| | Cells | Drug | Virus |
|---|---|---|---|
| 1. | + | - | - |
| 2. | + | + | - |
| 3. | + | - | + |
| 4. | + | + | + |

In experiments 2 and 4 the final concentrations of test compounds were 1, 10, 100 and 500 µg/ml. Either azidothymidine (AZT) or dideoxyinosine (ddI) was included as a positive drug control. Test compounds were dissolved in DMSO and diluted into tissue culture medium so that the final DMSO concentration did not exceed 1.5% in any case. DMSO was added to all control wells at an appropriate concentration.

Following the addition of virus, cells were incubated at 37°C in a humidified, 5% CO₂ atmosphere for 7 days. Test compounds could be added on days 0, 2 and 5 if desired. On day 7, post-infection, the cells in each well were resuspended and a 100µl sample of each cell suspension was removed for assay. A 20µL volume of a 5 mg/ml solution of 3-(4,5-dimethylthiazol-2-yl)-2,5-diphenyltetrazolium bromide (MTT) was added to each 100µL cell suspension, and the cells were incubated for 4 hours at 27°C in a 5% CO₂ environment. During this incubation, MTT is metabolically reduced by living cells resulting in the production in the cell of a colored formazan product. To each sample was added 100µl of 10% sodium dodecylsulfate in 0.01 N HCl to lyse the cells, and samples were incubated overnight. The absorbance at 590 nm was determined for each sample using a Molecular Devices microplate reader. Absorbance values for each set of wells is compared to assess viral control infection, uninfected control cell response as well as test compound by cytotoxicity and antiviral efficacy.

**TABLE 19**

| Compound | Inhibition |
|---|---|
| 1. Butanediamide, N¹-[3-[[[(2,2-dimethyl)propyl]carbonyl] (3-methylbutyl)amino]-2-hydroxy-1-(phenylmethyl)propyl]-2-[(2-quinolinylcarbonyl)amino]-, [1S-[1R*(2R*),2S*]] | 100% |

Following the procedures set forth above, the following compounds were also prepared:
Carbamic acid, [3-[[(4-amino-1,4-dioxo-2-[(2-quinolinylcarbonyl)amino]butyl]amino]-2-hydroxy-4-phenylbutyl][(4-fluorophenyl)methyl]-,1,1-dimethylethyl ester, [2R-[2R*,3S*(S*)]]-
Carbamic acid, [3-[[(4-amino-1,4-dioxo-2-[(2-quinolinylcarbonyl)amino]butyl]amino]-2-hydroxy-4-phenylbutyl][(3-methylbutyl)]-,1,1-dimethylethyl ester, [2R-[2R*,3S*(S*)]]-
Carbamic acid, [3-[[(4-amino-1,4-dioxo-2-[(2-quinolinylcarbonyl)amino]butyl]amino]-2-hydroxy-4-phenylbutyl][(2-methylpropyl)]-,1,1-dimethylethyl ester, [2R-[2R*,3S*(S*)]]-
Carbamic acid, [3-[[(4-amino-1,4-dioxo-2-[(2-quinolinylcarbonyl)amino]butyl]amino]-2-hydroxy-4-phenylbutyl][(4-pyridylmethyl)]-,1,1-dimethylethyl ester, [2R-[2R*,3S*(S*)]]-

The compounds of the present invention are effective antiviral compounds and, in particular, are effective retroviral inhibitors as shown above. Thus, the subject compounds are effective HIV protease inhibitors. It is contemplated that the subject compounds will also inhibit other viruses such as HIV, human T-cell leukemia virus, respiratory syncitial virus, hepadnavirus, cytomegalovirus and picornavirus.

The compounds of the present invention can be used in the form of salts derived from inorganic or organic acids. These salts include but are not limited to the following: acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glucoheptanoate, glycerophosphate, hemisulfate, heptanoate, hexanoate, fumarate, hydrochloride, hydrobromide, hydroiodide, 2-hydroxy-ethanesulfonate, lactate, maleate, methanesulfonate, nicotinate, 2-naphthalenesulfonate, oxalate, palmoate, pectinate, persulfate, 3-phenylpropionate, picrate, pivalate, propionate, succinate, tartrate, thiocyanate, tosylate, mesylate and undecanoate. Also, the basic nitrogen-containing groups can be quaternized with such agents as lower alkyl halides, such as methyl, ethyl, propyl, and butyl chloride, bromides, and iodides; dialkyl sulfates like dimethyl, diethyl, dibutyl, and diamyl sulfates, long chain halides such as decyl, lauryl, myristyl and stearyl chlorides, bromides and iodides, aralkyl halides like benzyl and phenethyl bromides, and others. Water or oil-soluble or dispersible products are thereby obtained.

Examples of acids which may be employed to form pharmaceutically acceptable acid addition salts include such inorganic acids as hydrochloric acid, sulphuric acid and phosphoric acid and such organic acids as oxalic acid, maleic acid, succinic acid and citric acid. Other examples include salts with alkali metals or alkaline earth metals, such as sodium, potassium, calcium or magnesium or with organic bases.

Total daily dose administered to a host in single or divided doses may be in amounts, for example, from 0.001 to 10 mg/kg body weight daily and more usually 0.01 to 1 mg. Dosage unit compositions may contain such amounts of submultiples thereof to make up the daily dose.

The amount of active ingredient that may be combined with the carrier materials to produce a single dosage form will vary depending upon the host treated and the particular mode of administration.

It will be understood, however, that the specific dose level for any particular patient will depend upon a variety of factors including the activity of the specific compound employed, the age, body weight, general health, sex, diet, time of administration, route of administration, rate of excretion, drug combination, and the severity of the particular disease undergoing therapy.

The compounds of the present invention may be administered orally, parenterally, by inhalation spray, rectally, or topically in dosage unit formulations containing conventional nontoxic pharmaceutically acceptable carriers, adjuvants, and vehicles as desired. Topical administration may also involve the use of transdermal administration such as transdermal patches or iontophoresis devices. The term parenteral as used herein includes subcutaneous injections, intravenous, intramuscular, intrasternal injection, or infusion techniques.

Injectable preparations, for example, sterile injectable aqueous or oleaginous suspensions may be formulated according to the known art using suitable dispersing or wetting agents and suspending agents. The sterile injectable preparation may also be a sterile injectable solution or suspension in a nontoxic parenterally acceptable diluent or solvent, for example, as a solution in 1,3-butanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

Suppositories for rectal administration of the drug can be prepared by mixing the drug with a suitable nonirritating excipient such as cocoa butter and polyethylene glycols which are solid at ordinary temperatures but liquid at the rectal temperature and will therefore melt in the rectum and release the drug.

Solid dosage forms for oral administration may include capsules, tablets, pills, powders, and granules. In such solid dosage forms, the active compound may be admixed with at least one inert diluent such as sucrose lactose or starch. Such dosage forms may also comprise, as in normal practice, additional substances other than inert diluents, e.g., lubricating agents such as magnesium stearate. In the case of capsules, tablets, and pills, the dosage forms may also comprise buffering agents. Tablets and pills can additionally be prepared with enteric coatings.

Liquid dosage forms for oral administration may include pharmaceutically acceptable emulsions, solutions, suspensions, syrups, and elixirs containing inert diluents commonly used in the art, such as water. Such compositions may also comprise adjuvants, such as wetting agents, emulsifying and suspending agents, and sweetening, flavoring, and perfuming agents.

While the compounds of the invention can be administered as the sole active pharmaceutical agent, they can also be used in combination with one or more immunomodulators, antiviral agents or other antiinfective agents. When administered as a combination, the therapeutic agents can be formulated as separate compositions which are given at the same time or different times, or the therapeutic agents can be given as a single composition.

The preceding examples can be repeated with similar success by substituting the generically or specifically described reactants and/or operating conditions of this invention for those used in the preceding examples.

## Claims

1. Compound represented by the formula 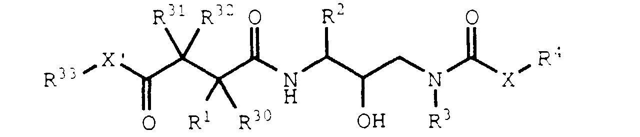 or a pharmaceutically acceptable salt thereof, wherein R¹ represents hydrogen, alkyl of 1 to 4 carbon atoms, aralkyl or hydroxyl radicals;
R³⁰, R³¹ and R³² represent radicals as defined for R¹, or one of R¹ and R³⁰ together with one of R³¹ and R³² and the carbon atoms to which they are attached form a cycloalkyl radical; or R³⁰ and R³² together with the carbon atoms to which they are attached form a three to six-membered cycloalkyl radical;
R² represents alkyl, aryl, cycloalkyl, cycloalkylalkyl or aralkyl radicals, which radicals are optionally substituted with halogen, -OR⁹ or SR⁹ radicals, wherein R⁹ represents hydrogen or alkyl radicals;
X represents O or CH(R¹⁷), wherein R¹⁷ represents hydrogen or alkyl radicals;
R³ represents alkyl radical of 2 to 5 carbon atoms and R⁴ represents alkyl or cycloalkyl radicals; or R³ represents heteroaralkyl, alkyl of 2 to 5 carbon atoms, benzyl, para-fluorobenzyl, para-methoxybenzyl, para-methylbenzyl, 2-naphthylmethyl or cyclohexylmethyl radicals and R⁴ represents t-butyl; or provided X represents CHR¹⁷, R³ and R⁴ independently represent alkyl or hydroxyalkyl radicals;
X' represents NR³⁴ or O; and
R³³ and R³⁴ independently represent hydrogen and radicals as defined for R³; or provided X' represents NR³⁴, R³³ and R³⁴ together with a nitrogen atom to which they are bonded represent heterocyclyl or heteroaryl radicals; or provided X' represents O, R³³ represents hydrogen, alkyl or aralkyl radicals; and
wherein alkyl, alone or in combination, is a straight-chain or branched-chain alkyl radical containing from 1 to 8 carbon atoms; cycloalkyl, alone or in combination, is an cyclic alkyl radical containing from 3 to 8 carbon atoms; aryl, alone or in combination, is an unsubstituted phenyl or naphthyl radical, or phenyl or naphthyl radical substituted with a C₁-C₈ alkyl, C₁-C₈ alkoxy, halogen, hydroxy or amino radical; heterocycloalkyl, alone or in combination, is pyrrolidinyl, piperidinyl, piperazinyl, morpholinyl, thiamorpholinyl, tetrahydroquinolinyl or tetrahydroisoquinolinyl radicals, which radicals are optionally substituted on a substitutable carbon atom with halogen, C₁-C₈ alkyl, C₁-C₈ alkoxy or oxo, on a ring secondary nitrogen atom with C₁-C₈ alkyl, aryl-C₁-C₈-alkoxycarbonyl, C₁-C₈ alkanoyl, phenyl or phenyl-C₁-C₈-alkyl, or on a ring tertiary nitrogen atom by oxido; and heteroaryl, alone or in combination, is pyrrolyl, imidazolyl, pyrazolyl, pyridyl, pyrazinyl, pyrimidinyl, furyl, thienyl, triazolyl, oxazolyl, thiazolyl, indolyl, quinolinyl, isoquinolinyl, quinoxalinyl, β-carbolinyl, benzofuranyl or benzimidazolyl radicals, which radicals are optionally substituted on a substitutable carbon atom with halogen, C₁-C₈ alkyl, C₁-C₈ alkoxy or oxo, on a ring secondary nitrogen atom with C₁-C₈ alkyl, aryl-C₁-C₈-alkoxycarbonyl, C₁-C₈ alkanoyl, phenyl or phenyl-C₁-C₈-alkyl, or on a ring tertiary nitrogen atom by oxido.

2. Compound of Claim 1 wherein
R¹ and R³¹ are both hydrogen radical and R³⁰ and R³² are both methyl radical; or R³⁰ is hydrogen radical and R¹, R³¹ and R³² are each methyl radical; or R³⁰, R³¹ and R³² are each hydrogen radical and R¹ is methyl radical; or R¹ and
R³¹ are each hydrogen radical and R³⁰ and R³² together with the carbon atoms to which they are attached form a three to six-membered cycloalkyl radical;
R² represents alkyl, cycloalkylalkyl or aralkyl radicals optionally substituted with halogen, -OR⁹ or SR⁹ radicals, wherein R⁹ represents hydrogen or alkyl radicals; and
R³ represents heteroaralkyl, alkyl of 2 to 5 carbon atoms, benzyl, para-fluorobenzyl, para-methoxybenzyl, para-methylbenzyl, 2-naphthylmethyl or cyclohexylmethyl radicals and R⁴ represents t-butyl; and
R³³ and R³⁴ independently represent hydrogen and radicals as defined for R³; or provided X' represents O, R³³ represents hydrogen, alkyl or aralkyl radicals.

3. Compound of Claim 2 wherein
R¹ and R³¹ are both hydrogen radical and R³⁰ and R³² are both methyl radical; or R³⁰ is hydrogen radical and R¹, R³¹ and R³² are each methyl radical; or R³⁰, R³¹ and R³² are each hydrogen radical and R¹ is methyl radical;
R² represents CH₃SCH₂CH₂-, iso-butyl, n-butyl, benzyl, 4-fluorobenzyl, 2-naphthylmethyl or cyclohexylmethyl radicals;
R³ represents n-propyl, i-butyl, neo-pentyl, n-pentyl i-amyl, n-butyl, cyclohexylmethyl, benzyl, para-fluorobenzyl, para-methoxybenzyl, para-methylbenzyl or 2-naphthylmethyl radicals;
R⁴ represents t-butyl radical; and
X' represents O and R³³ represents hydrogen or alkyl radicals.

4. Compound of Claim 1 wherein R¹ represents hydrogen, methyl, ethyl, benzyl, phenylpropyl or hydroxyl radicals;
R² represents alkyl, cycloalkylalkyl or aralkyl radicals;
R³ represents alkyl of 2 to 5 carbon atoms or heteroaralkyl radical; and
R⁴ represents tert-butyl radical.

5. A pharmaceutical composition comprising a compound according to any of claims 1 to 4 and a pharmaceutically acceptable carrier.

6. Use of a compound defined in any of claims 1 to 4 for the manufacture of a medicament for inhibiting retroviral protease.

7. Use according to claim 6 wherein the retroviral protease is HIV protease.

8. Use of a compound defined in any of claims 1 to 4 for the manufacture of a medicament for treatment of a retroviral infection.

9. Use according to claim 8 wherein the retroviral infection is an HIV infection.

10. Use of a compound defined in any of claims 1 to 4 for the manufacture of a medicament for the treatment of AIDS.

## Patentansprüche

1. Verbindung, dargestellt durch die Formel 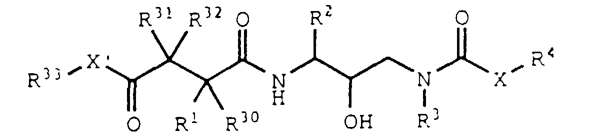 oder ein pharmazeutisch annehmbares Salz davon, worin R¹ Wasserstoff-, Alkyl- mit 1 bis 4 Kohlenstoffatomen, Aralkyl- oder Hydroxylreste darstellt;
R³⁰, R³¹ und R³² Reste wie für R¹ definiert darstellen oder eins von R¹ und R³⁰ zusammen mit einem von R³¹ und R³² und den Kohlenstoffatomen, an die sie gebunden sind, einen Cycloalkylrest bilden; oder R³⁰ und R³² zusammen mit den Kohlenstoffatomen, an die sie gebunden sind, einen drei- bis sechsgliedrigen Cycloalkylrest bilden;
R² Alkyl-, Aryl-, Cycloalkyl-, Cycloalkylalkyl- oder Aralkylreste darstellt, welche Reste gegebenenfalls mit Halogen-, -OR⁹- oder SR⁹-Resten substituiert sind, worin R⁹ Wasserstoff oder Alkylreste darstellt;
X für O oder CH(R¹⁷) steht, worin R¹⁷ Wasserstoff oder Alkylreste darstellt;
R³ Alkylrest mit 2 bis 5 Kohlenstoffatomen darstellt und R⁴ Alkyl- oder Cycloalkylreste darstellt; oder R³ Heteroaralkyl-, Alkyl- mit 2 bis 5 Kohlenstoffatomen, Benzyl-, Parafluorbenzyl-, Paramethoxybenzyl-, Paramethylbenzyl-, 2-Naphthylmethyl- oder Cyclohexylmethylreste darstellt und R⁴ t-Butyl darstellt; oder vorausgesetzt dass X für CHR¹⁷ steht, R³ und R⁴ unabhängig Alkyl- oder Hydroxyalkylreste darstellen;
X' für NR³⁴ oder O steht; und
R³³ und R³⁴ unabhängig Wasserstoff und wie für R³ definierte Reste darstellen, oder vorausgesetzt dass X' für NR³⁴ steht, R³³ und R³⁴ zusammen mit einem Stickstoffatom, an das sie gebunden sind, Heterocyclyl- oder Heteroarylreste darstellen; oder vorausgesetzt dass X' für O steht, R³³ Wasserstoff, Alkyl- oder Aralkylreste darstellt; und
worin Alkyl allein oder in Kombination für einen geradkettigen oder verzweigtkettigen Alkylrest steht, der 1 bis 8 Kohlenstoffatome enthält; Cycloalkyl allein oder in Kombination für einen cyclischen Alkylrest steht, der 3 bis 8 Kohlenstoffatome enthält; Aryl allein oder in Kombination für einen nicht substituierten Phenyl- oder Naphthylrest, oder Phenyl- oder Naphthylrest, substituiert mit einem C₁-C₈-Alkyl-, C₁-C₈-Alkoxy-, Halogen-, Hydroxy- oder Aminorest steht; Heterocycloalkyl allein oder in Kombination für Pyrrolidinyl-, Piperidinyl-, Piperazinyl-, Morpholinyl-, Thiamorpholinyl-, Tetrahydrochinolinyl- oder Tetrahydroisochinolinylreste steht, welche Reste gegebenenfalls an einem substituierbaren Kohlenstoffatom mit Halogen, C₁-C₈-Alkyl, C₁-C₈-Alkoxy oder Oxo, an einem sekundären Ring-Stickstoffatom mit C₁-C₈-Alkyl, Aryl-C₁-C₈-alkoxycarbonyl, C₁-C₈-Alkanoyl, Phenyl oder Phenyl-C₁-C₈-alkyl, oder an einem tertiären Ring-Stickstoffatom durch Oxido substituiert sind; und Heteroaryl allein oder in Kombination für Pyrrolyl-, Imidazolyl- Pyrazolyl-, Pyridyl-, Pyrazinyl-, Pyrimidiniyl-, Furyl-, Thienyl-, Triazolyl-, Oxazolyl-, Thiazolyl-, Indolyl-, Chinolinyl-, Isochinolinyl-, Chinoxalinyl-, β-Carbolinyl-, Benzofuranyl- oder Benzimidazolylreste steht, welche Reste gegebenenfalls an einem substituierbaren Kohlenstoffatom mit Halogen, C₁-C₈-Alkyl, C₁-C₈-Alkoxy oder Oxo, an einem sekundären Ring-Stickstoffatom mit C₁-C₈-Alkyl, Aryl-C₁-C₈-alkoxycarbonyl, C₁-C₈-Alkanoyl, Phenyl oder Phenyl-C₁-C₈-alkyl, oder an einem tertiären Ring-Stickstoffatom durch Oxido substituiert sind.

2. Verbindung von Anspruch 1, worin
R¹ und R³¹ beide einen Wasserstoffrest darstellen und R³⁰ und R³² beide einen Methylrest darstellen; oder R³⁰ einen Wasserstoffrest darstellt und R¹, R³¹ und R³² jeweils einen Methylrest darstellen; oder R³⁰, R³¹ und R³² jeweils einen Wasserstoffrest darstellen und R¹ einen Methylrest darstellt; oder R¹ und R³¹ jeweils einen Wasserstoffrest darstellen und R³⁰ und R³² zusammen mit den Kohlenstoffatomen, an die sie gebunden sind, einen drei- oder sechsgliedrigen Cycloalkylrest darstellen;
R² für Alkyl-, Cycloalkylalkyl- oder Aralkylreste steht, gegebenenfalls substituiert mit Halogen-, -OR⁹- oder SR⁹-Resten, worin R⁹ Wasserstoff- oder Alkylreste darstellt; und
R³ für Heteroaralkyl-, Alkyl- mit 2 bis 5 Kohlenstoffatomen, Benzyl-, Parafluorbenzyl-, Paramethoxybenzyl-, Paramethylbenzyl-, 2-Naphthylmethyl- oder Cyclohexylmethylreste steht und R⁴ t-Butyl darstellt; und
R³³ und R³⁴ unabhängig Wasserstoff und Reste, wie für R³ definiert darstellen; oder vorausgesetzt dass X' für O steht, R³³ Wasserstoff-, Alkyl- oder Aralkylreste darstellt.

3. Verbindung von Anspruch 2, worin
R¹ und R³¹ beide einen Wasserstoffrest darstellen und R³⁰ und R³² beide einen Methylrest darstellen; oder R³⁰ einen Wasserstoffrest darstellt und R¹, R³¹ und R³² jeweils einen Methylrest darstellen; oder R³⁰, R³¹ und R³² jeweils einen Wasserstoffrest darstellen und R¹ einen Methylrest darstellt;
R² für CH₃SCH₂CH₂-, Isobutyl-, n-Butyl-, Benzyl-, 4-Fluorbenzyl-, 2-Naphthylmethyl- oder Cyclohexylmethylreste steht;
R³ für n-Propyl-, i-Butyl-, Neopentyl-, n-Pentyl-, i-Amyl-, n-Butyl-, Cyclohexylmethyl-, Benzyl-, Parafluorbenzyl-, Paramethoxybenzyl-, Paramethylbenzyl- oder 2-Naphthylmethylreste steht.
R⁴ einen t-Butylrest darstellt; und
X' für O steht und R³³ Wasserstoff- oder Alkylreste darstellt.

4. Verbindung von Anspruch 1, worin R¹ Wasserstoff-, Methyl-, Ethyl-, Benzyl-, Phenylpropyl- oder Hydroxylreste darstellt;
R² Alkyl-, Cycloalkylalkyl- oder Aralkylreste darstellt;
R³ Alkyl- mit 2 bis 5 Kohlenstoffatomen oder einen Heteroaralkylrest darstellt; und
R⁴ einen tert-Butylrest darstellt.

5. Pharmazeutische Zusammensetzung, umfassend eine Verbindung gemäss einem der Ansprüche 1 bis 4 und einen pharmazeutisch annehmbaren Träger.

6. Verwendung einer in einem der Ansprüche 1 bis 4 definierten Verbindung für die Herstellung einer Arznei zum Hemmen retroviraler Protease.

7. Verwendung gemäß Anspruch 6, worin die retrovirale Protease HIV-Protease ist.

8. Verwendung einer in einem der Ansprüche 1 bis 4 definierten Verbindung für die Herstellung einer Arznei zur Behandlung einer retroviralen Infektion.

9. Verwendung gemäß Anspruch 8, worin die retrovirale Infektion eine HIV-Infektion ist.

10. Verwendung einer in einem der Ansprüche 1 bis 4 definierten Verbindung für die Herstellung einer Arznei zur Behandlung von AIDS.

## Revendications

1. Composé représenté par la formule : 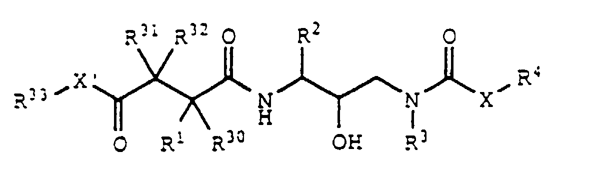 ou un sel pharmaceutiquement acceptable de celui-ci, où R¹ représente un atome d'hydrogène, des radicaux alkyle comportant de 1 à 4 atomes de carbone, aralkyle ou hydroxyle ;
R³⁰, R³¹ et R³² représentent des radicaux tels que définis pour R¹, ou un des R¹ et R³⁰, avec un des R³¹ et R³² et les atomes de carbone auxquels ils sont attachés, forment un radical cycloalkyle ; ou R³⁰ et R³², avec les atomes de carbone auxquels ils sont attachés, forment un radical cycloalkyle à 3 à 6 chaînons ;
R² représente des radicaux alkyle, aryle, cycloalkyle, cycloalkylalkyle ou aralkyle, lesquels radicaux sont facultativement substitués par un atome d'halogène, des radicaux -OR⁹ ou SR⁹, où R⁹ représente un atome d'hydrogène ou des radicaux alkyle ;
X représente O ou CH(R¹⁷), où R¹⁷ représente un atome d'hydrogène ou des radicaux alkyle ;
R³ représente un radical alkyle comportant de 2 à 5 atomes de carbone, et R⁴ représente des radicaux alkyle ou cycloalkyle ; ou R³ représente des radicaux hétéroaralkyle, alkyle comportant de 2 à 5 atomes de carbone, benzyle, parafluorobenzyle, paraméthoxybenzyle, parméthylbenzyle, 2-naphtylméthyle ou cyclohexylméthyle, et R⁴ représente le radical t-butyle ; ou sous réserve que X représente CHR¹⁷,
R³ et R⁴ représentent indépendamment des radicaux alkyle ou hydroxyalkyle ;
X' représente NR³⁴ ou O ; et
R³³ et R³⁴ représentent indépendamment un atome d'hydrogène et des radicaux tels que définis pour R³ ; ou sous réserve que X' représente NR³⁴, R³³ et R³⁴, avec l'atome d'azote auquel ils sont liés, représentent des radicaux hétérocyclyle ou hétéroaryle ; ou sous réserve que X' représente O, R³³ représente un atome d'hydrogène, des radicaux alkyle ou aralkyle ; et
où un radical alkyle, seul ou en combinaison, est un radical alkyle à chaîne linéaire ou à chaîne ramifiée, contenant de 1 à 8 atomes de carbone ; un radical cycloalkyle, seul ou en combinaison, est un radical alkyle cyclique contenant de 3 à 8 atomes de carbone ; un radical aryle, seul ou en combinaison, est un radical phényle ou naphtyle non substitué, ou un radical phényle ou naphtyle substitué par un radical alkyle en C₁-C₈, alcoxy en C₁-C₈, un atome d'halogène, un radical hydroxy ou amino ; un radical hétérocycloalkyle, seul ou en combinaison, est un radical pyrrolidinyle, pipéridinyle, pipérazinyle, morpholinyle, thiamorpholinyle, tétrahydroquinolinyle ou tétrahydroisoquinolinyle, lesquels radicaux sont facultativement substitués, sur un atome de carbone pouvant être substitué, par un atome d'halogène, un radical alkyle en C₁-C₈, alcoxy en C₁-C₈, ou oxo, sur un atome d'azote secondaire du cycle par un radical alkyle en C₁-C₈, aryl (alcoxy en C₁-C₈) carbonyle, alcanoyle en C₁-C₈, phényle, ou phényl(alkyle en C₁-C₈), ou sur un atome d'azote tertiaire du cycle par un radical oxido ; et un radical hétéroaryle, seul ou en combinaison, est un radical pyrrolyle, imidazolyle, pyrazolyle, pyridyle, pyrazinyle, pyrimidinyle, furyle, thiényle, triazolyle, oxazolyle, thiazolyle, indolyle, quinolinyle, isoquinolinyle, quinoxalinyle, β-carbolinyle, benzofuranyle ou benzimidazolyle, lesquels radicaux sont facultativement substitués sur un atome de carbone pouvant être substitué, par un atome d'halogène, un radical alkyle en C₁-C₈, alcoxy en C₁-C₈, ou oxo, sur un atome d'azote secondaire du cycle par un radical alkyle en C₁-C₈, aryl-(alcoxy en C₁-C₈)carbonyle, alcanoyle en C₁-C₈, phényle, ou phényl(alkyle en C₁-C₈), ou sur un atome d'azote tertiaire du cycle par un radical oxido.

2. Composé selon la revendication 1, dans lequel
R¹ et R³¹ représentent tous les deux un atome d'hydrogène et R³⁰ et R³² représentent tous les deux un radical méthyle ; ou
R³⁰ représente un atome d'hydrogène et R¹, R³¹ et R³² représentent chacun un radical méthyle ; ou R³⁰, R³¹ et R³² représentent chacun un atome d'hydrogène, et R¹ représente un radical méthyle ; ou R¹ et R³¹ représentent chacun un atome d'hydrogène, et R³⁰ et R³², avec les atomes de carbone auxquels ils sont attachés, forment un radical cycloalkyle à 3 à 6 chaînons ;
R² représente des radicaux alkyle, cycloalkylalkyle ou aralkyle facultativement substitués par des atomes d'halogène, des radicaux -OR⁹ ou SR⁹, où R⁹ représente un atome d'hydrogène ou un radical alkyle ; et
R³ représente des radicaux hétéroaralkyle, alkyle comportant de 2 à 5 atomes de carbone, benzyle, parafluorobenzyle, para-méthoxybenzyle, para-méthylbenzyle, 2-naphtylméthyle ou cyclohexylméthyle, et R⁴ représente le radical t-butyle ; et
R³³ et R³⁴ représentent indépendamment un atome d'hydrogène et des radicaux tels que définis pour R³ ; ou sous réserve que X' représente O, R³³ représente un atome d'hydrogène, des radicaux alkyle ou aralkyle.

3. Composé selon la revendication 2, dans lequel
R¹ et R³¹ représentent tous les deux un atome d'hydrogène et R³⁰ et R³² représentent tous les deux un radical méthyle ; ou
R³⁰ représente un atome d'hydrogène et R¹, R³¹ et R³² représentent chacun un radical méthyle ; ou R³⁰, R³¹ et R³² représentent chacun un atome d'hydrogène, et R¹ représente un radical méthyle ;
R² représente CH₃SCH₂CH₂-, des radicaux isobutyle, n-butyle, benzyle, 4-fluorobenzyle, 2-naphtylméthyle ou cyclohexylméthyle, ;
R³ représente des radicaux n-propyle, i-butyle, néopentyle, n-pentyle, i-amyle, n-butyle, cyclohexylméthyle, benzyle, parafluorobenzyle, para-méthoxybenzyle, paraméthylbenzyle ou 2-naphtylméthyle ;
R⁴ représente le radical t-butyle ; et
X' représente O et R³³ représente un atome d'hydrogène ou des radicaux alkyle.

4. Composé selon la revendication 1, dans lequel R¹ représente un atome d'hydrogène, des radicaux méthyle, éthyle, benzyle, phénylpropyle ou hydroxyle ; R² représente des radicaux alkyle, cycloalkylalkyle ou aralkyle ;
R³ représente un radical alkyle comportant de 2 à 5 atomes de carbone, ou un radical hétéroaralkyle ; et
R⁴ représente le radical tert-butyle.

5. Composition pharmaceutique comprenant un composé selon l'une quelconque des revendications 1 à 4, et un support pharmaceutiquement acceptable.

6. Utilisation d'un composé selon l'une quelconque des revendications 1 à 4, pour la fabrication d'un médicament pour inhiber une protéase rétrovirale.

7. Utilisation selon la revendication 6, dans laquelle la protéase rétrovirale est la protéase du HIV.

8. Utilisation d'un composé selon l'une quelconque des revendications 1 à 4, pour la fabrication d'un médicament pour le traitement d'une infection rétrovirale.

9. Utilisation selon la revendication 8, dans laquelle l'infection rétrovirale est une infection par le HIV.

10. Utilisation d'un composé selon l'une quelconque des revendications 1 à 4, pour la fabrication d'un médicament pour le traitement du SIDA.
